# EUROPEAN PATENT APPLICATION

(11) **EP 4 292 547 A1**
(43) Date of publication of application: **20.12.2023**
(21) Application number: 22752301.6
(22) Date of filing: 09.02.2022
(51) Int. Cl.: A61B 17/12, A61B 18/14

(54) **ABLATION SYSTEM**

(30) Priority: 09.02.2021 CN 202110177082; 08.06.2021 CN 202110638975; 10.12.2021 CN 202111510577
(71) Applicant: Hangzhou Dinova EP Technology Co., Ltd., Hangzhou, Zhejiang 310052 (CN)
(72) Inventor: WANG, Kun, Hangzhou, Zhejiang 310052 (CN); LI, Jianmin, Hangzhou, Zhejiang 310052 (CN); CHEN, Zhixin, Hangzhou, Zhejiang 310052 (CN); YOU, Yan, Hangzhou, Zhejiang 310052 (CN); QIU, Jiaming, Hangzhou, Zhejiang 310052 (CN)
(74) Representative: Petraz, Gilberto Luigi
(86) International application number: PCT/CN2022/075719
(87) International publication number: WO 2022/171141

(57) **Abstract**

An ablation system (3300) includes a support (3310), a delivery device (3320) configured to deliver the support (3310) to a target tissue, and an ablation element (3330) including a first conductive portion (3331) and a second conductive portion (3332). The first conductive portion (3331) is provided on the support (3310). The second conductive portion (3332) is provided on the support (3310) or the delivery device (3320) or independently from the support (3310) and the delivery device (3320). Both the first conductive portion (3331) and the second conductive portion (3332) are electrically connected to an external pulse ablation source, and the first conductive portion (3331) and the second conductive portion (3332) are configured to transmit pulse ablation energy with different polarities to the target tissue.

## Description

### TECHNICAL FIELD

The present disclosure refers to the technical field of interventional medical devices, in particular, to an ablation system.

### DESCRIPTION OF THE PRIOR ART

Atrial fibrillation (AF) is the most common sustained arrhythmia. The occurrence of AF increases with age, with a prevalence of 10% of people over 75 years old. During atrial fibrillation, the frequency of atrial pacing reaches 300-600 beats/min, and the heartbeat is often rapid and irregular, sometimes with a heart rate up to 100-160 beats/min, which is faster than a normal heart rate, with arrhythmia and poor atrial contraction. Atrial fibrillation often increases the risk of many potentially lethal complications, including thromboembolic stroke, dilated cardiomyopathy, and congestive heart failure. Common AF symptoms such as palpitations, chest pain, dyspnea, fatigue, and dizziness can also affect quality of life. People with atrial fibrillation have a five-fold increase in morbidity and a two-fold increase in mortality than normal people.

Tissue ablation is usually used to treat various cardiac arrhythmias, including atrial fibrillation. To treat cardiac arrhythmias, an ablation catheter is used to perform ablation to alter tissue properties, for example, to prevent abnormal electrical propagation and/or disrupt abnormal electrical conduction through cardiac tissue. Most existing ablation catheters are based on thermal ablation, such as radiofrequency ablation, laser ablation, microwave ablation, thermal substance ablation, etc..

However, ablation devices based on thermal ablation perform ablation on the tissue by heating. During the ablation, the ablation electrodes of the ablation device are required to contact the wall closely. In the case where the ablation electrodes contact the wall loosely, the success rate of ablation decreases.

### SUMMARY OF THE DISCLOSURE

The purpose of the embodiments of the present invention is to provide an ablation system,
including:
a support,
a delivery device, a distal end of the delivery device is connected to the support, and the delivery device is configured to deliver the support to a target tissue; and
an ablation element, including a first conductive portion and a second conductive portion, the first conductive portion provided on the support, the second conductive portion provided on the support or the delivery device or independently from the support and the delivery device, both the first conductive portion and the second conductive portion electrically connected to an external pulse ablation source, and the first conductive portion and the second conductive portion are configured to transmit pulse ablation energy with different polarities to the target tissue to achieve tissue ablation.

The beneficial effects of the ablation system provided by the present invention are: compared with the prior art, the ablation system of the present invention can transmit pulse ablation energy with different polarities to the target tissue through the first conductive portion and the second conductive portion cooperating with each other, to achieve pulse ablation of the target tissue. The first conductive portion is provided on the support, and the second conductive portion is provided on the support or the delivery device or independently from the support and the delivery device.

The ablation system of the present invention, based on pulse ablation, utilizes a pulsed electric field to cause irreversible electrical breakdown of the cell membrane (irreversible electroporation), causing cell apoptosis and thus achieving non-thermal ablation of cells, without heat sink effect. The pulse sequence generates less heat and does not need saline irrigation for cooling, which can effectively reduce the occurrence of gas explosion, eschar and thrombus. The treatment time for pulse ablation is short. The treatment time of applying a set of pulse sequences is less than 1 minute, and the whole ablation time is generally no more than 5 minutes. Moreover, since different tissues have different response thresholds to a pulsed electric field, it is possible to perform ablation on the myocardium without disturbing other surrounding tissues, thereby avoiding accidentally injuring surrounding tissues of the pulmonary vein. In addition, compared with other energies, pulse energy does not require heat conduction to ablate the deep tissue, and all target tissue cells distributed in the area with a certain electric intensity will suffer from electroporation, which reduces the requirement for the contact pressure of the ablation element during ablation. Therefore, even if the ablation device does not contact the inner wall of the tissue closely during the ablation, the ablation effect will not be affected.

### BRIEF DESCRIPTION OF DRAWINGS

In order to describe the technical solutions according to the embodiments of the present disclosure more clearly, the drawings accompanying the embodiments are briefly introduced below. Apparently, the drawings below only illustrate some embodiments of the present invention, and those skilled in the art can obtain other drawings based on the drawings below without creative work.
FIG. 1 is a schematic view of a left atrial appendage occlusion and ablation device according to the first embodiment of the present disclosure;
FIG. 2 is a schematic view of a left atrial appendage occlusion and ablation device according to the second embodiment of the present disclosure;
FIG. 3 is a schematic view of a left atrial appendage occlusion and ablation device according to the third embodiment of the present disclosure;
FIG. 4 is a schematic view of a left atrial appendage occlusion and ablation device according to the fourth embodiment of the present disclosure;
FIG. 5 is a schematic view of a left atrial appendage occlusion and ablation device according to the fifth embodiment of the present disclosure;
FIG. 6 is a schematic view of a left atrial appendage occlusion and ablation device according to the sixth embodiment of the present disclosure;
FIG. 7 is a schematic view of a left atrial appendage occlusion and ablation device according to the seventh embodiment of the present disclosure;
FIG. 8 is a schematic view of a left atrial appendage occlusion and ablation device according to the eighth embodiment of the present disclosure;
FIG. 9 is a schematic view of a left atrial appendage occlusion and ablation device according to the ninth embodiment of the present disclosure;
FIG. 10 is a schematic view of a left atrial appendage occlusion and ablation device according to the tenth embodiment of the present disclosure;
FIG. 11 is a schematic view of a left atrial appendage occlusion and ablation device according to the eleventh embodiment of the present disclosure;
FIG. 12 is a schematic view of a left atrial appendage occlusion and ablation device according to the twelfth embodiment of the present disclosure;
FIG. 13 is a schematic view of a left atrial appendage occlusion and ablation device according to the thirteenth embodiment of the present disclosure;
FIG. 14 is a schematic view of a left atrial appendage occlusion and ablation device according to the fourteenth embodiment of the present disclosure;
FIG. 15 is a schematic view of a left atrial appendage occlusion and ablation device according to the fifteenth embodiment of the present disclosure;
FIG. 16 is a schematic view of the spaced ablation zones of the ablation element according to an embodiment;
FIG. 17 is a schematic view of the partially overlapping ablation zones of the ablation element according to an embodiment;
FIG. 18 is a schematic view of a left atrial appendage occlusion and ablation device according to the sixteenth embodiment of the present disclosure;
FIG. 19 is a schematic view of a left atrial appendage occlusion and ablation device according to the seventeenth embodiment of the present disclosure;
FIG. 20 is a schematic view of a left atrial appendage occlusion and ablation device according to the eighteenth embodiment of the present disclosure;
FIG. 21 is a schematic view of a left atrial appendage occlusion and ablation device according to the nineteenth embodiment of the present disclosure;
FIG. 22 is a schematic view of a left atrial appendage occlusion and ablation device according to the twentieth embodiment of the present disclosure;
FIG. 23 is a schematic view of a left atrial appendage occlusion and ablation device according to the twenty-first embodiment of the present disclosure;
FIG. 24 is a schematic view of a left atrial appendage occlusion and ablation device according to the twenty-second embodiment of the present disclosure;
FIG. 25 is a schematic view of a left atrial appendage occlusion and ablation device according to the twenty-third embodiment of the present disclosure;

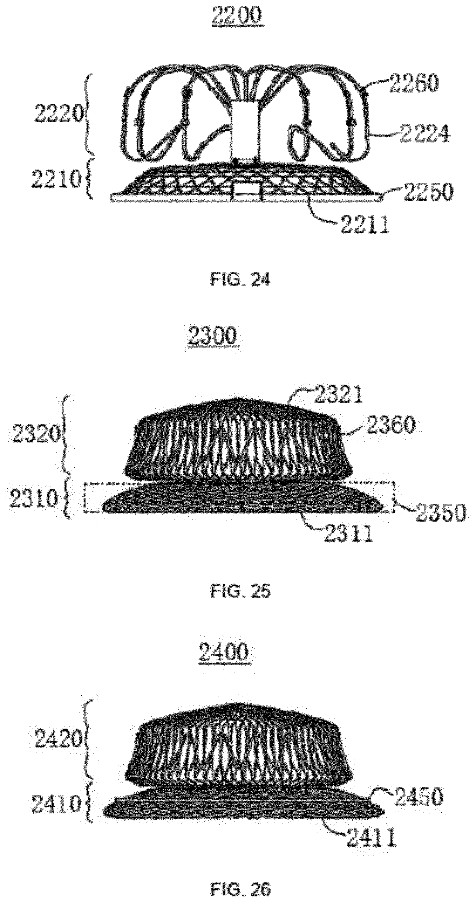

FIG. 25 is a schematic view of a left atrial appendage occlusion and ablation device according to the twenty-fourth embodiment of the present disclosure;

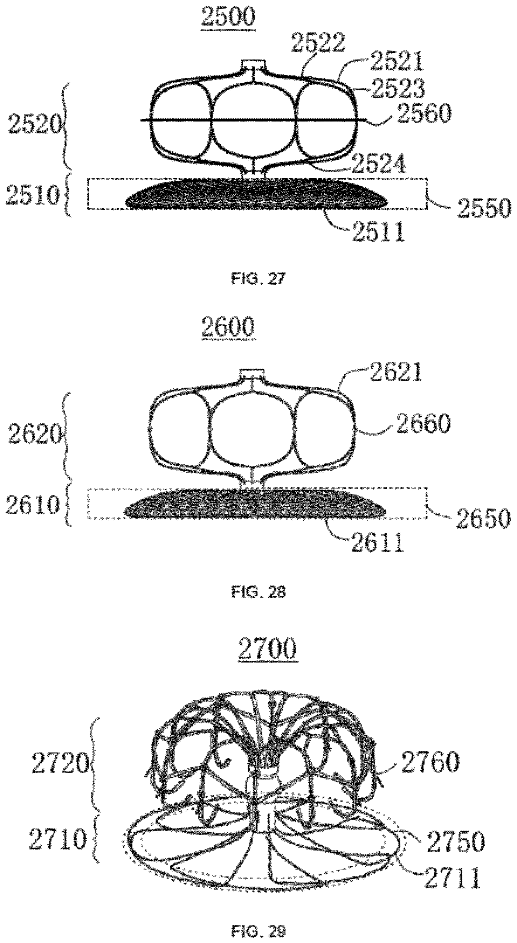

FIG. 27 is a schematic view of a left atrial appendage occlusion and ablation device according to the twenty-fifth embodiment of the present disclosure;
FIG. 28 is a schematic view of a left atrial appendage occlusion and ablation device according to the twenty-sixth embodiment of the present disclosure;
FIG. 29 is a schematic view of a left atrial appendage occlusion and ablation device according to the twenty-seventh embodiment of the present disclosure;

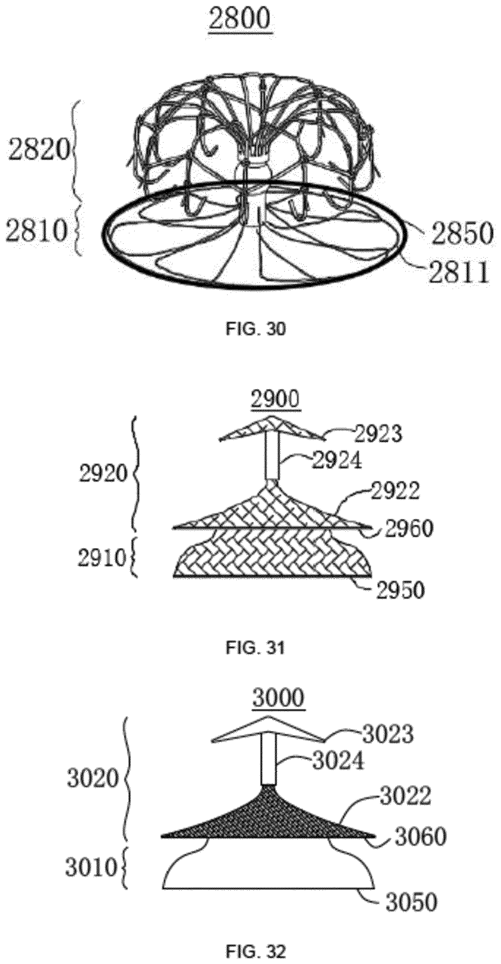

FIG. 30 is a schematic view of a left atrial appendage occlusion and ablation device according to the twenty-eighth embodiment of the present disclosure;
FIG. 31 is a schematic view of a left atrial appendage occlusion and ablation device according to the twenty-ninth embodiment of the present disclosure;
FIG. 32 is a schematic view of a left atrial appendage occlusion and ablation device according to the thirtieth embodiment of the present disclosure;

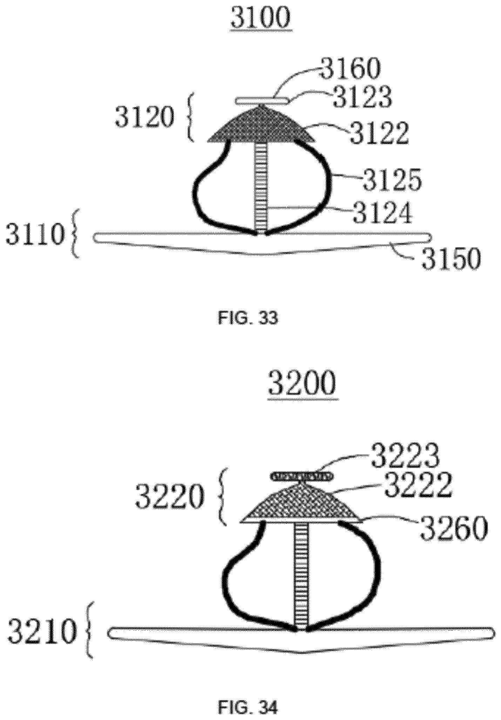

FIG. 33 is a schematic view of a left atrial appendage occlusion and ablation device according to the thirty-first embodiment of the present disclosure;
FIG. 34 is a schematic view of a left atrial appendage occlusion and ablation device according to the thirty-second embodiment of the present disclosure;

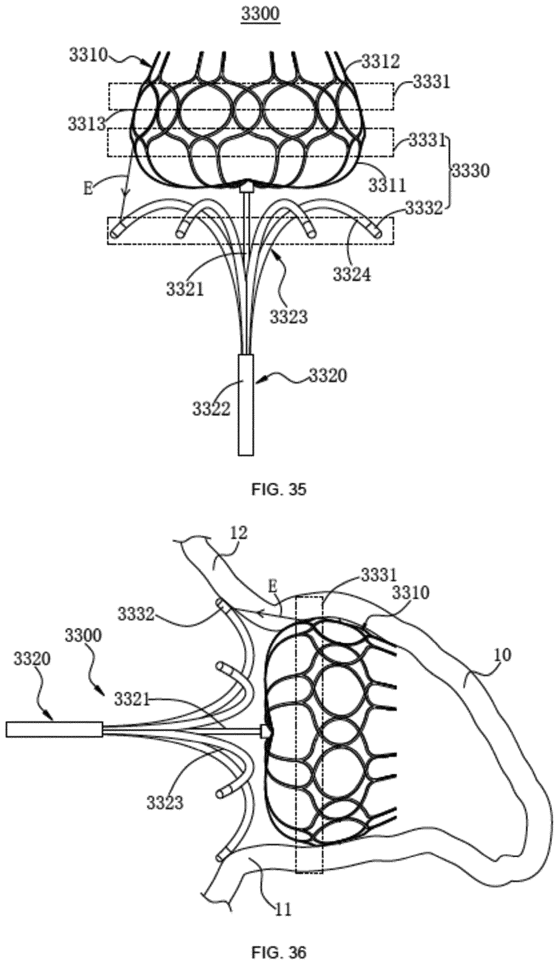

FIG. 35 is a schematic view of an ablation system according to the thirty-third embodiment of the present disclosure;
FIG. 36 is a schematic view of the ablation system shown in FIG. 35 in operation;

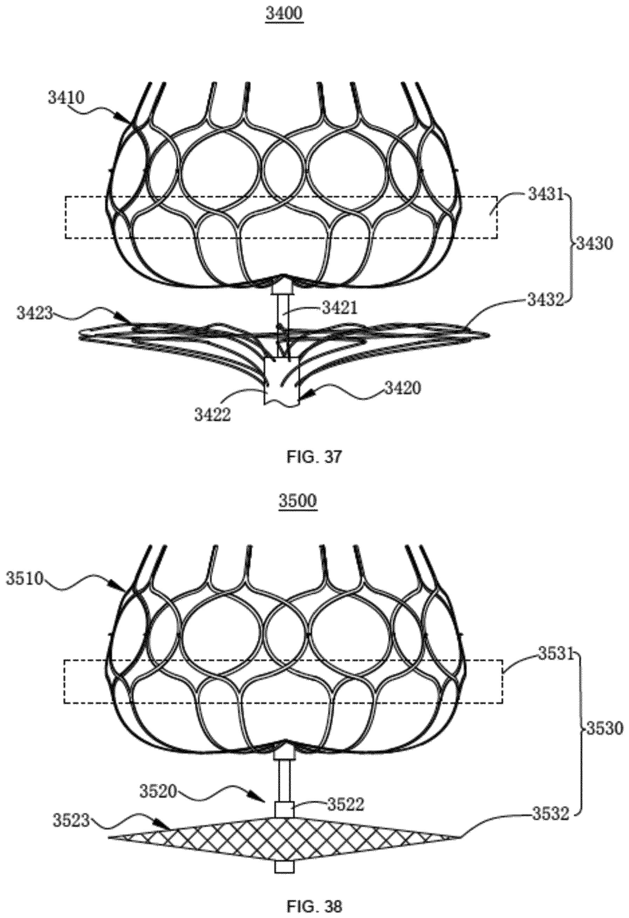

FIG. 37 is a schematic view of the ablation system according to the thirty-fourth embodiment of the present disclosure;
FIG. 38 is a schematic view of the ablation system according to the thirty-fifth embodiment of the present disclosure;

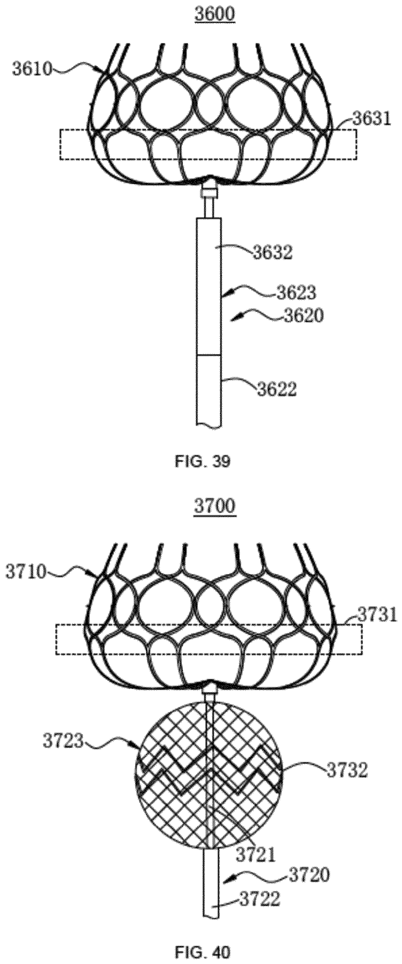

FIG. 39 is a schematic view of the ablation system according to the thirty-sixth embodiment of the present disclosure;
FIG. 40 is a schematic view of the ablation system according to the thirty-seventh embodiment of the present disclosure;

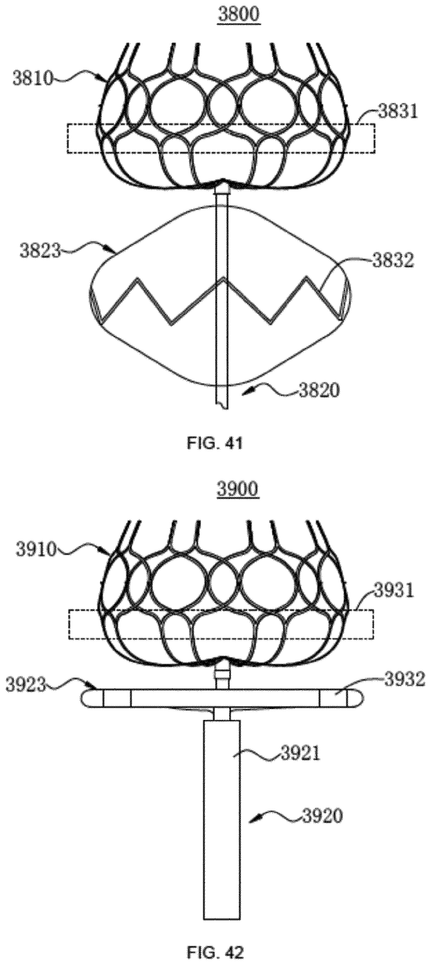

FIG. 41 is a schematic view of the ablation system according to the thirty-eighth embodiment of the present disclosure;
FIG. 42 is a schematic view of the ablation system according to the thirty-ninth embodiment of the present disclosure;

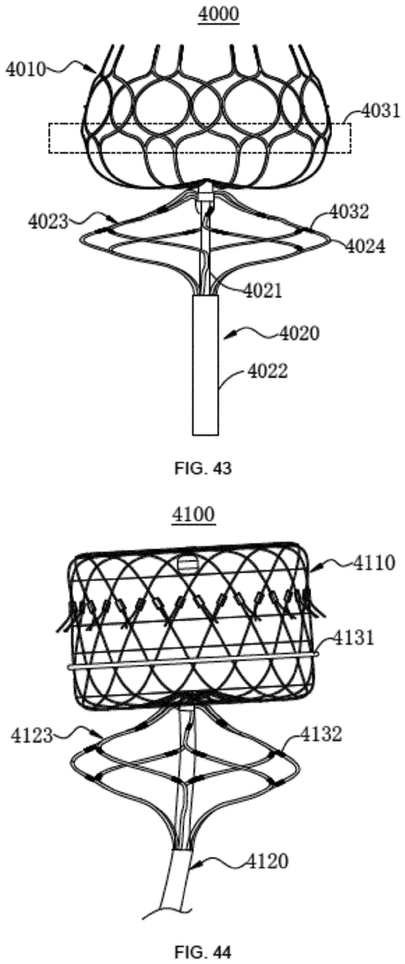

FIG. 43 is a schematic view of the ablation system according to the fortieth embodiment of the present disclosure;
FIG. 44 is a schematic view of the ablation system according to the forty-first embodiment of the present disclosure;

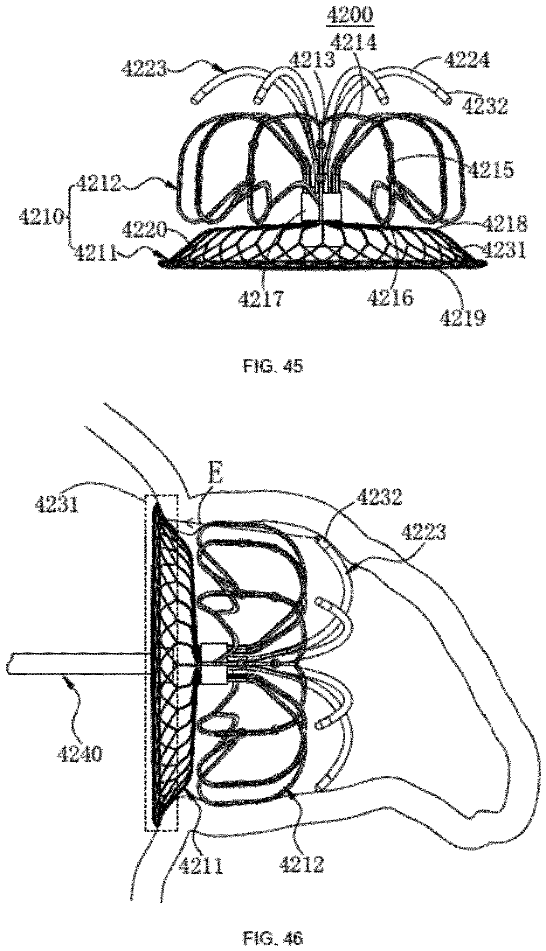

FIG. 45 is a schematic view of the ablation system according to the forty-second embodiment of the present disclosure;
FIG. 46 is a schematic view of the ablation system shown in FIG. 45 in operation;

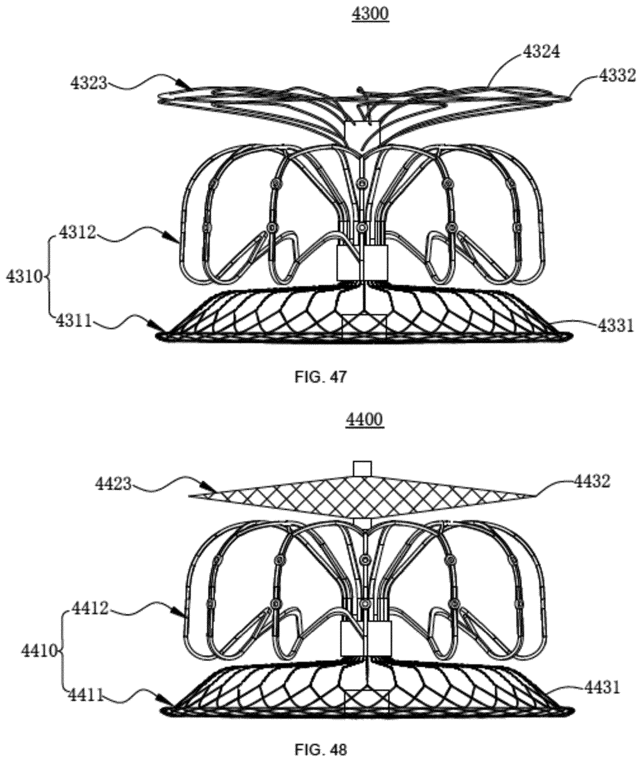

FIG. 47 is a schematic view of the ablation system according to the forty-third embodiment of the present disclosure;
FIG. 48 is a schematic view of the ablation system according to the forty-fourth embodiment of the present disclosure;

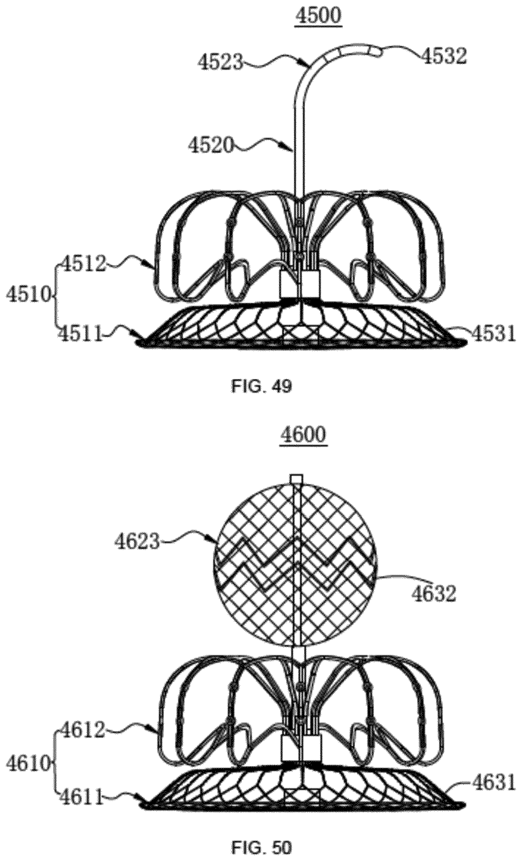

FIG. 49 is a schematic view of the ablation system according to the forty-fifth embodiment of the present disclosure;
FIG. 50 is a schematic view of the ablation system according to the forty-sixth embodiment of the present disclosure;

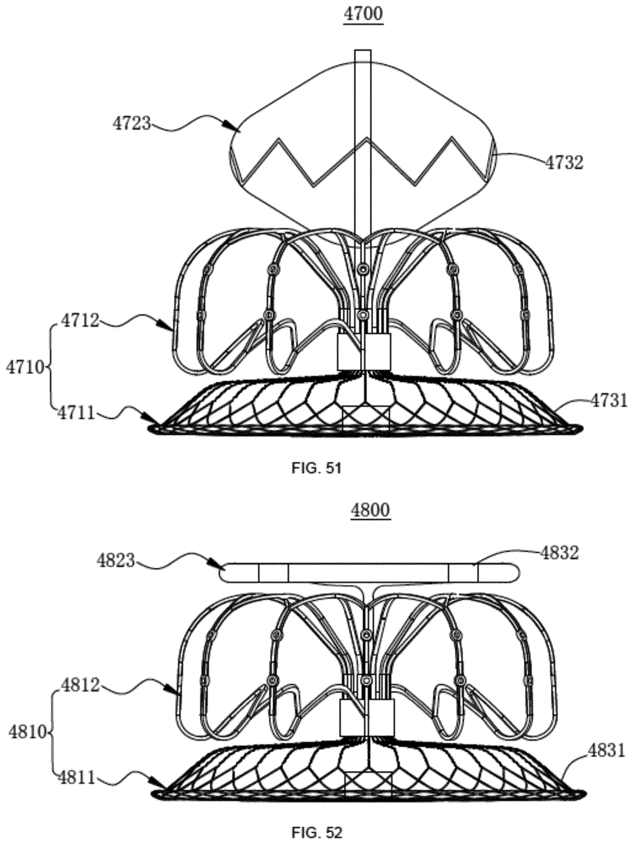

FIG. 51 is a schematic view of the ablation system according to the forty-seventh embodiment of the present disclosure;
FIG. 52 is a schematic view of the ablation system according to the forty-eighth embodiment of the present disclosure;

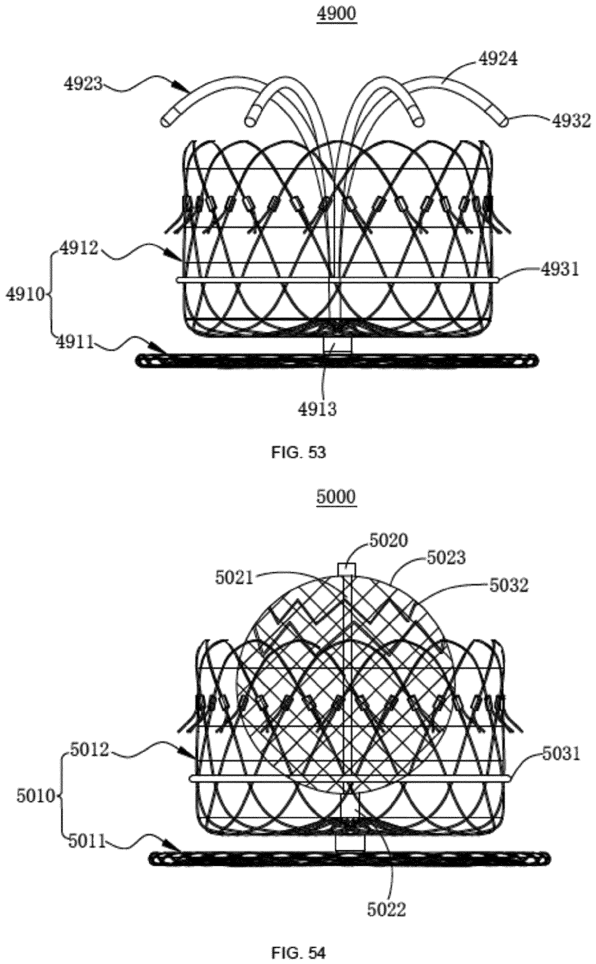

FIG. 53 is a schematic view of the ablation system according to the forty-ninth embodiment of the present disclosure;
FIG. 54 is a schematic view of the ablation system according to the fiftieth embodiment of the present disclosure;

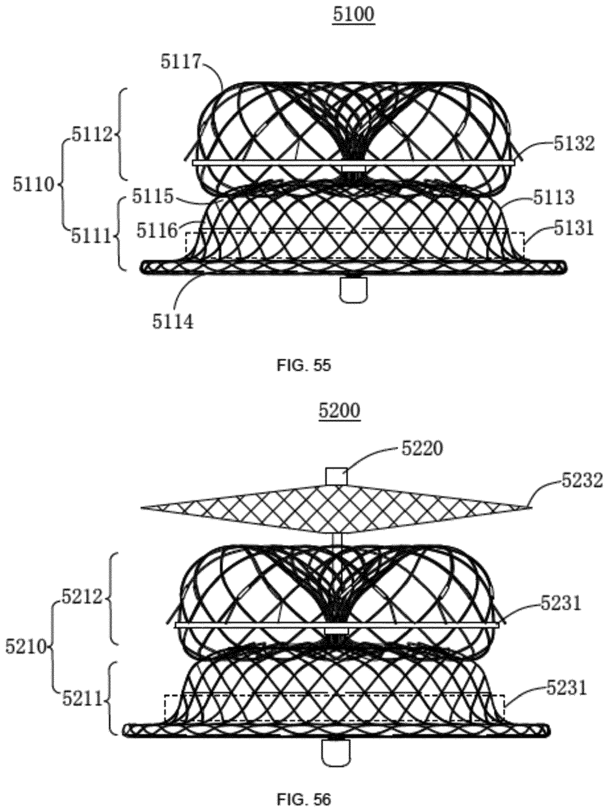

FIG. 55 is a schematic view of the ablation system according to the fifty-first embodiment of the present disclosure;
FIG. 56 is a schematic view of the ablation system according to the fifty-second embodiment of the present disclosure;

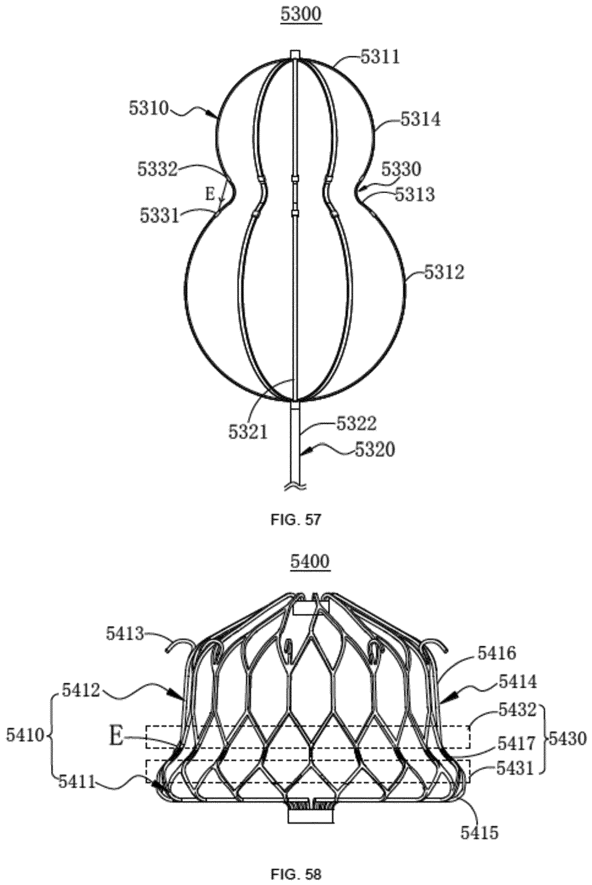

FIG. 57 is a schematic view of the ablation system according to the fifty-third embodiment of the present disclosure; and
FIG. 58 is a schematic view of the ablation system according to the fifty-fourth embodiment of the present disclosure.

### DESCRIPTION OF EMBODIMENTS

In order to make the technical problems to be solved by the present invention, the technical solutions and the technical effects apparent, the present invention will be further described in detail below in conjunction with the accompanying drawings and embodiments. It should be noted that the specific embodiments described here are only used to explain the present invention, and are not intended to limit the present invention.

It should be noted that when an element is referred to as being "fixed with" or "provided on" another element, it may be directly fixed with or provided on another element or be indirectly fixed with or provided on another element. When an element is referred to as being "connected to" another element, it can be directly connected to another element or indirectly connected to another element.

It should be noted that the terms "center", "longitudinal", "transverse", "length", "width", "thickness", "upper", "lower", "front", "rear", "left", "right", "vertical", "horizontal", "top", "bottom", "inner", "outer", etc., for indicating the orientation or positional relationship are based on the orientation or positional relationship shown in the drawings, and are only for the convenience and simplification of the description, but not to indicate or imply that the device or element referred to must have a specific orientation, be constructed and operate in a specific orientation, and thus should not be construed as limiting the present invention. As used herein, the term "and/or" includes any and all combinations of one or more of the listed items.

In addition, the terms "first" and "second" are used for descriptive purposes only, and cannot be interpreted as indicating or implying relative importance or the quantity of referred features. Thus, a feature defined with "first" or "second" may explicitly or implicitly include one or more of such features. In the description of the present disclosure, "plurality" means two or more, unless otherwise specifically defined.

### Definitions:

Left atrial appendage ostium: the position from the left atrium into the left atrial appendage.

Proximal and distal ends: in the field of interventional medical devices, the proximal end usually refers to the end of a medical device implanted in a human or animal body close to the operator, and the distal end usually refers to the end away from the operator. The same applies to the definition of proximal and distal ends of any part of the medical device. Without departing from the concept of the present invention, the specific technical features in the following embodiments may be replaced with each other.

Axial and radial directions: "axial direction" usually refers to the length direction of the medical device being delivered, and "radial direction" usually refers to the direction perpendicular to the "axial direction" of the medical device. The same applies to the definition of axial and radial directions of any part of the medical device.

Insulation treatment: providing an insulating layer on the surface of a part of a component to insulate the part of the component. Specifically, the methods of insulation treatment include: providing insulating coating at the portion where insulation treatment is required, including but not limited to Parylene coating, PTFE (Poly-tetrafluoroethylene) coating, PI (Polyimide, polyimide) coating; or, providing an insulation film covering the portion where insulation treatment is required, including but not limited to FEP (Fluorinated-ethylene-propylene), PU (polyurethane), ETFE (ethylene-tetra-fluoroethylene), PFA (Polyfluoroalkoxy), PTFE, PEEK (poly-ether-ether-ketone), silicone; or, surrounding the portion where insulation treatment is required with an insulating sleeve, the material of which includes but is not limited to FEP, PU, ETFE, PFA, PTFE, PEEK, and silica gel. In some embodiments, at least one insulation treatment method described above may be applied at the portion where insulation treatment is required.

### First Embodiment

The ablation system according to the embodiment disclosed here delivers an ablation device through a delivery device to the target tissue by percutaneous puncture, including but not limited to a specific site in the heart, such as the pulmonary vein, the left atrial appendage, or non-PV trigger foci combined with typical atrial flutter such as superior vena cava, coronary sinus ostium, etc., to perform ablation so as to achieve electrical isolation. It can be understood that the tissue site to be ablated is not limited to be located in the heart, but may be located in other body tissues, which is not limited here.

The ablation system according to the embodiment disclosed here may be an ablation system for occlusion and ablation of left atrial appendage, or for occlusion and ablation of other tissues, or an ablation catheter, not for occlusion of tissue, but for ablation of target tissue, i.e., an ablation system only for ablation of tissue. The ablation system according to the embodiment disclosed here includes a support, a delivery device, and an ablation element. The distal end of the delivery device is connected to the support for delivering the support to the target tissue. The ablation element includes a first conductive portion and a second conductive portion. The first conductive portion is provided on the support. The second conductive portion is provided on the support or the delivery device, or independent from the support and the delivery device. Both the first conductive portion and the second conductive portion are electrically connected to an external pulse ablation source, and the first conductive portion and the second conductive portion are configured to transmit pulse ablation energy with different polarities to the target tissue for tissue ablation.

The support includes a radially compressible and expandable frame or a balloon. The frame can be made by weaving or cutting and have a plurality of meshes. The balloon can be filled with a medium to adjust the radial size. In the following, the frame is taken as an example for description.

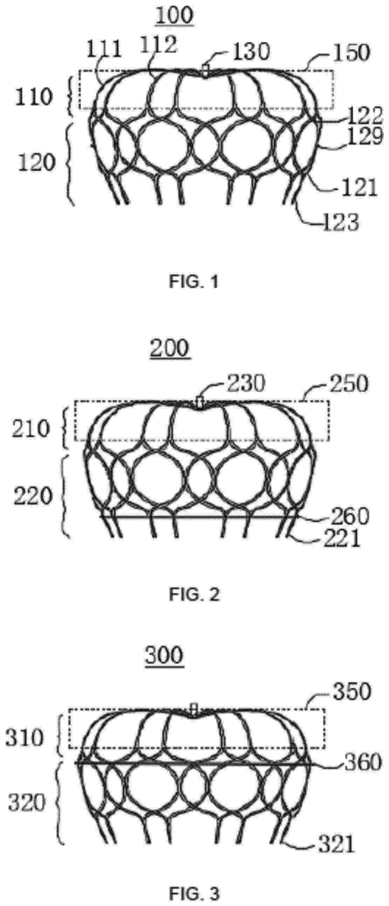

Referring to FIG. 1, the ablation system according to this embodiment includes a left atrial appendage occlusion and ablation device 100 and a delivery device. The left atrial appendage occlusion and ablation device 100 is configured for occlusion and electrical ablation of the left atrial appendage. The left atrial appendage occlusion and ablation device 100 includes a support and a first conductive portion provided on the support. In this embodiment, the support can be made by weaving or cutting and is configured as a frame with a plurality of meshes. The second conductive portion is provided on the support of the left atrial appendage occlusion and ablation device 100, or on the delivery device, or independently from the support and the delivery device (that is, the second conductive portion is provided outside the support and the delivery device, for example, the second conductive portion may be a metal plate configured to contact the patient's body surface during the ablation process). In this embodiment, the first conductive portion is electrically connected to the external pulse ablation instrument through the delivery device. The target tissue is the left atrial appendage in myocardial tissue.

The frame includes a sealing part 110 and an anchoring part 120 connected to each other. The sealing part 110 is configured to cover the ostium of the left atrial appendage. The anchoring part 120 is configured to be fixed on the tissue wall of the left atrial appendage. Both the sealing part 110 and the anchoring part 120 are radially compressible and expandable structures, and any part of the sealing part 110 and the anchoring part 120 can be made by weaving or cutting. In this embodiment, the sealing part 110 is provided at the proximal end of the anchoring part 120. In some other embodiments, the anchoring part may be provided at the peripheral edge of the sealing part.

Specifically, the sealing part 110 includes a first frame 111, the anchoring part includes a second frame 121, and the first frame 111 and the second frame 121 are connected to each other. The sealing part 110 is provided with an ablation element for transmitting ablation energy. Specifically, the first conductive portion 150 of the ablation element is configured for transmitting ablation energy.

The left atrial appendage occlusion and ablation device 100 in this embodiment has a single-disc structure, that is, the sealing part 110 and the anchoring part 120 of the left atrial appendage occlusion and ablation device 100 are configured on the same disc. The frames of the sealing part 110 and the anchoring part 120 on the peripheral edge areas thereof are configured to contact the tissue of the left atrial appendage.

The sealing part 110 is provided on the proximal side of the anchoring part 120. The proximal surface of the sealing part 110 is configured to be released at the ostium of the left atrial appendage. The peripheral edge area of the first frame 111 is configured to contact the ostium of the left atrial appendage and the tissue wall of the inner cavity of the left atrial appendage. Especially, the portion of the first frame 111 with the maximum radial dimension is configured to contact the tissue wall of the inner cavity of the left atrial appendage. Optionally, at least one layer of flow blocking film is provided on the inside or outside of the left atrial appendage occlusion and ablation device 100 to prevent the thrombus from flowing out, so as to prevent the thrombus inside the left atrial appendage from flowing out to the left atrium. The anchoring part 120 is configured to be released in the inner cavity of the left atrial appendage, and the circumferential sidewall of the second frame of the anchoring part 120 is configured for providing radial support force on the inner wall of the left atrial appendage and being fixed in the inner cavity of the left atrial appendage. Optionally, as shown in FIG. 1, the second frame includes a plurality of anchoring barbs 129 arranged on its surface, and the anchoring barbs 129 are tilted and arranged in the circumferential direction of the frame in a radial pattern, so that after the anchoring part 120 is released, the anchoring barbs 129 can engage with the tissue around the inner cavity of the left atrial appendage to improve the anchoring stability of the anchoring part 120.

As shown in FIG. 1, the anchoring part 120 has a distal opening so that the left atrial appendage occlusion and ablation device 100 assumes a cup-shaped structure.

The sealing part 110 is provided with a first conductive portion 150 for transmitting ablation energy. In some embodiments, at least part of the first conductive portion 150 can be configured to collect physiological signals of the tissue.

In this embodiment, at least part of the first frame 111 is configured as the first conductive portion 150. Specifically, the entire first frame 111 is configured as the first conductive portion 150. The first conductive portion 150 is configured to transmit ablation energy. In some embodiments, the first conductive portion 150 can be further configured to collect physiological signals of the tissue, such as physiological electrical signals, so as to realize signal mapping before and after ablation.

The tissue at the ostium of the left atrial appendage has a smooth surface and a regular shape relative to the tissue around the inner cavity of the left atrial appendage, so that the sealing part 110 can contact the tissue at the ostium of the left atrial appendage closely. The entire first frame of the sealing part 110 is configured as the first conductive portion for ablating the tissue at the ostium of the left atrial appendage, so that the first conductive portion 150 can contact the wall closely, facilitating the ablation on the tissue at the ostium of the left atrial appendage.

In some embodiments, part of the first frame 111 is configured as the first conductive portion 150. Specifically, the first frame 111 includes an edge area and a central area. The central area is closer to the central axis of the sealing part 110 than the edge area. The central axis passes through the central area. The portion of the sealing part 110 with the maximum radial dimension is located on the peripheral edge area. The first conductive portion 150 is provided on the peripheral edge area of the sealing part 110, so that the first conductive portion 150 can contact or be close to the tissue of the left atrial appendage. Further, in some embodiments, the first conductive portion 150 is provided on the area of the first frame 111 with the maximum radial dimension.

The first conductive portion 150 is provided on the edge area of the first frame 111, or the first conductive portion 150 is further provided on the area of the first frame 111 with the maximum radial dimension, thereby reducing the area where the ablation energy is released and concentrating the ablation energy at the first conductive portion 150 with a small discharge area, which is beneficial to increase the ablation depth.

Specifically, the sealing part 110 is made of conductive material, which can be made of superelastic metal material with good biocompatibility, such as stainless steel, nickel-titanium alloy, cobalt-chromium alloy among others. Since the left atrial appendage occlusion and ablation device 100 has a single-disc structure in this embodiment, i.e., the sealing part 110 and the anchoring part 120 are configured on the same disc, in order to simplify the manufacturing process, the sealing part 110 and the anchoring part 120 are made with the same material by the same method. The left atrial appendage occlusion and ablation device 100 in FIG. 1 is made of a nickel-titanium alloy tube by laser cutting. It would be appreciated that the left atrial appendage occlusion and ablation device 100 can be made of other metal materials with good biocompatibility, or be made by a method other than cutting, such as weaving.

In this embodiment, the anchoring part 120 is not configured to ablate the tissue. The surface of the second frame 121 of the anchoring part 120 is subjected to insulation treatment, and the specific method for the insulation treatment can refer to the above-mentioned description. In this embodiment, the sealing part 110 is provided with a first conductive portion 150 for ablation of tissue, that is, the left atrial appendage occlusion and ablation device 100 can use the first conductive portion 150 to ablate the tissue.

As shown in FIG. 1, the left atrial appendage occlusion and ablation device 100 includes a first conductive connection part 130 provided on the first frame 111. The first conductive connection part 130 is electrically connected between the first conductive portion 150 and an external ablation energy source which may be a pulse ablation instrument configured to output pulse ablation electric energy for the left atrial appendage occlusion and ablation device 100 to perform ablation.

Pulse ablation utilizes a pulsed electric field to cause irreversible electrical breakdown of the cell membrane (irreversible electroporation), causing cell apoptosis and thus achieving non-thermal ablation of cells, without heat sink effect. The pulse sequence generates less heat and does not need saline irrigation for cooling, which can effectively reduce the occurrence of gas explosion, eschar and thrombus. The treatment time for pulse ablation is short. The treatment time of applying a set of pulse sequences is less than 1 minute, and the whole ablation time is generally no more than 5 minutes. Moreover, since different tissues have different response thresholds to a pulsed electric field, it is possible to perform ablation on the myocardium without disturbing other surrounding tissues, thereby avoiding accidentally injuring surrounding tissues of the pulmonary vein. In addition, compared with other energies, pulse energy does not require heat conduction to ablate the deep tissue, and all target tissue cells distributed in the area with a certain electric intensity will suffer from electroporation, which reduces the requirement for the contact pressure of the ablation element during ablation. Therefore, even if the ablation device does not contact the inner wall of the tissue closely during the ablation, the ablation effect will not be affected.

The first conductive connection part 130 is disposed at the proximal end of the sealing part 110, and is preferably made of conductive material to transmit electric energy, such as stainless steel, nickel-titanium alloy, cobalt-chromium alloy among others. Preferably, the proximal end of the first frame 111 is converged in the first conductive connection part 130. The first conductive connection part 130 is further configured to connect the delivery device. The distal end of the delivery device is mechanically and electrically connected to the first conductive connection part 130, and the proximal end of the delivery device is electrically connected with the external ablation energy source.

In some embodiments, a second conductive portion is provided outside the left atrial appendage occlusion and ablation device 100 for cooperating with the first conductive portion 150, and the second conductive portion is configured to transmit ablation energy. In some embodiments, at least part of the second conductive portion can be used for collecting physiological signals of the tissue. The first conductive portion is electrically isolated from the second conductive portion, and the first conductive portion and the second conductive portion can be configured to transmit pulse ablation energy with different polarities to the tissue of the left atrial appendage, so as to realize tissue ablation. The difference between the first conductive portion and the second conductive portion lies in the polarity. During the ablation process, the conductive portion with one polarity is the first conductive portion, and the conductive portion with the other polarity is the second conductive portion. The first conductive portion is provided on the support.

The second conductive portion can be provided on the delivery device, for example, on a catheter of the delivery device configured to connect the first conductive connection part 130, or a movable member (such as an ablation catheter) of the delivery device which can move relative to the left atrial appendage occlusion and ablation device 100 and is configured to be released from the distal end of the delivery device. An ablation electric field can be generated between the first conductive portion 150 and the second conductive portion for ablating the tissue of the left atrial appendage. The second conductive portion can be released on the proximal side, the inner cavity or the distal side of the left atrial appendage occlusion and ablation device 100. In some embodiments, the second conductive portion is provided independently from the left atrial appendage occlusion and ablation device 100 and the delivery device. The first conductive portion 150 of the left atrial appendage occlusion and ablation device 100 is configured to cooperate with the second conductive portion provided outside the left atrial appendage occlusion and ablation device 100 and the delivery device, for example, the second conductive portion configured to contact the patient's body surface during the ablation process. The second conductive portion can be connected to the external pulse ablation instrument through the delivery device or other cables.

In some embodiments, in the case where the first frame 111 of the sealing part 110 is configured as the first conductive portion 150, the left atrial appendage occlusion and ablation device 100 is provided with a second conductive portion at the anchoring part 120. The second conductive portion may be part or the entire of the second frame of the anchoring part 120. The first conductive portion 150 is insulated from and connected with the second conductive portion.

More specifically, in the single-disc left atrial appendage occlusion and ablation device 100 shown in FIG. 1, at least part of the first frame 111 is configured as the first conductive portion, and at least part of the second frame 121 is configured as the second conductive portion. The first frame 111 is insulated from and connected with the second frame 121. For example, the first frame and the second frame can be connected with each other through an insulating material, such as insulating glue. The distal end of the first frame 111 and/or the proximal end of the second frame 121 are made of insulating material, or the distal end of the first frame 111 and/or the proximal end of the second frame 121 are connected with each other by a connecting piece made of insulating material.

In the case where the anchoring part 120 is provided with the second conductive portion, the left atrial appendage occlusion and ablation device 100 is further provided with a second conductive connection part electrically connected with the second conductive portion. The first conductive connection part 130 is electrically isolated from the second conductive connection part so that they can transmit ablation electric energy with different polarities. Specifically, the first conductive connection part 130 is insulated from and connected with the second conductive connection part. For example, an insulating material can be arranged between them. The second conductive connection part and the second conductive portion can be electrically connected to each other through a second conducting wire.

In the case where the anchoring part 120 is provided with the second conductive portion, while the left atrial appendage occlusion and ablation device 100 does not include the second conductive connection part, the second conductive portion is electrically connected to the delivery device through a second conducting wire. After ablation, the second conducting wire is separated from the delivery device, or the second conducting wire is separated from the second conductive portion, or the second conducting wire is controlled to be broken off. It would be appreciated that, the first conductive portion 150 and the delivery device can be connected to each other using the same connecting method for the second conductive portion and the delivery device, which will not be repeated here.

Compared with the tissue around the inner cavity of the left atrial appendage, the tissue at the ostium of the left atrial appendage has a smooth surface and a regular shape, which is convenient for the sealing part 110 to contact the wall. Part or the entire of the first frame 111 of the sealing part 110 is configured as the first conductive portion 150 for ablating the tissue at the ostium of the left atrial appendage so that the first conductive portion 150 can contact the wall closely, facilitating the ablation on the tissue at the ostium of the left atrial appendage.

Further, referring to FIG. 1 again, the first frame 111 includes a plurality of first sealing struts 112. Each first sealing strut 112 extends between the proximal end and the distal end. The proximal ends of the plurality of first sealing struts 112 are converged to the first conductive connection part 130. It can be understood that the proximal ends of the plurality of first sealing struts 112 are not necessarily limited to be converged to the first conductive connection part 130, but can be connected with other parts. The plurality of first sealing struts 112 extend around the first conductive connection part 130 in different directions, and the distal ends of the plurality of first sealing struts 112 are spaced apart from each other, so that the first frame 111 assumes a hollow structure with meshes.

The second frame 121 is formed by extending from the ends of the plurality of spaced first sealing struts 112 of the first frame 111, and includes first anchoring struts 122 and second anchoring struts 123. The first anchoring strut 122 is connected between the first sealing strut 121 and the second anchoring strut 123. The distal end of each first sealing strut 112 is connected to two different first anchoring struts 122, which extend in different directions. Within four first anchoring struts 122 connected to two adjacent first sealing struts 112, the middle sections of two adjacent first anchoring struts 122 connected to different first sealing struts 112 are closely connected to each other, where the anchoring barb 129 is provided. The distal ends of the two first anchoring struts 122 connected to the same first sealing strut 112 are connected to each other, which are connected to the proximal end of the second anchoring strut 123. The distal end of the second anchoring strut 123 extends toward the distal side. Adjacent second anchoring struts 123 are spaced apart from each other, that is, the distal ends of the second anchoring struts 123 are not connected or converged each other, thereby forming a structure with an opening at the distal end of the anchoring part 120, that is, the distal end of the second frame 121 and also the distal end of the left atrial appendage occlusion and ablation device 100 have an opening.

The plurality of first anchoring struts 122 define a plurality of meshes for the anchoring part 120, so as to improve the radial deformation ability of the anchoring part 120 and increase the friction between the anchoring part 120 and the tissue.

The anchoring barb 129 extends from the proximal end toward the distal end. The fixed end of the anchoring barb 129 is connected with the first anchoring strut 122, and the free end of the anchoring barb 129 tilts radially outwardly relative to the first anchoring strut 122, to facilitate the engagement with the tissue inside the left atrial appendage.

### Second Embodiment

Referring to FIG. 2, the ablation system according to this embodiment includes a left atrial appendage occlusion and ablation device 200, which is configured to occlude and ablate the left atrial appendage. The left atrial appendage occlusion and ablation device 200 includes a sealing part 210 disposed at the proximal side and an anchoring part 220 disposed at the distal side, and the sealing part 210 and the anchoring part 220 are connected to each other.

The main difference between the left atrial appendage occlusion and ablation device 200 according to this embodiment and the left atrial appendage occlusion and ablation device 100 is that a second conductive portion 260 is provided on the frame of the left atrial appendage occlusion and ablation device 200 for transmitting ablation energy. The second conductive portion 260 is provided on the anchoring part 220. The first conductive portion 250 and the second conductive portion 260 are electrically isolated from each other. The first conductive portion 250 and the second conductive portion 260 are configured to transmit pulse ablation energy with different polarities to the left atrial appendage. At least part of the first conductive portion 250 and/or the second conductive portion 260 can be configured to collect physiological signals of the tissue.

Specifically, the second conductive portion 260 is at least one electrode element additionally disposed on the surface of the anchoring part 220. The second conductive portion 260 is made of a conductive material with good biocompatibility, such as platinum, iridium, gold, silver and other medical metals that can be used for interventional therapy.

In this embodiment, the second conductive portion 260 includes one electrode element, and specifically, the electrode element is a ring electrode.

The ring electrode is an electrode wire or an electrode sheet, which extends in the circumferential direction of the anchoring part 220 and around the anchoring part 220 in a circle. The ring electrode is in a ring shape, such as a circular ring shape, an elliptical ring shape, or an irregular ring shape, so that a ring-like ablation zone can be provided.

As shown in FIG. 2, the ring electrode is arranged in a plane perpendicular to the central axis of the left atrial appendage occlusion and ablation device 200, that is, the portions of the ring electrode are located at the same axial level of the left atrial appendage occlusion and ablation device 200. In other embodiments, the ring electrode can be arranged in a plane inclined relative to the central axis of the left atrial appendage occlusion and ablation device 200.

As shown in FIG. 2, the second conductive portion 260 is disposed on the distal portion of the anchoring part 220 and cooperates with the first conductive portion 250 to ablate the tissue.

In some embodiments, the surface of the entire second frame 221 is subjected to insulation treatment. In some embodiments, the part of the second frame 221 that is not in contact with the second conductive portion 260 is subjected to insulation treatment, while the part of the second frame 221 that is in contact with the second conductive portion 260 does not need insulation treatment. In some embodiments, the part of the second frame 221 that is in contact with the second conductive portion 260 is subjected to insulation treatment, while the part of the second frame 221 that is not in contact with the second conductive portion 260 does not need insulation treatment or is selectively subjected to insulation treatment.

The two conductive portions are configured to transmit pulse ablation energy with different polarities, and an ablation electric field for ablating the target tissue can be generated between the two conductive portions. The ablation zone of each conductive portion is a zone where the intensity of the electric field generated around the conductive portion is greater than the intensity threshold of the target tissue, so that the target tissue within the ablation zone can be ablated. Specifically, the zone where the intensity of the electric field generated around the first conductive portion 250 is greater than the field intensity threshold of the target tissue is the first ablation zone, and the zone where the intensity of the electric field generated around the second conductive portion 260 is greater than the field intensity threshold of the target tissue is the second ablation zone.

The left atrial appendage occlusion and ablation device 200 is configured to ablate the left atrial appendage in the myocardial tissue. When the intensity of the electric field for pulse ablation of the left atrial appendage is lower than the field intensity threshold of the myocardial tissue, the ablation electric field cannot ablate the left atrial appendage, and the left atrial appendage has no physiological response. When the intensity of the electric field for pulse ablation of the left atrial appendage is higher than the field intensity threshold of the myocardial tissue, the pulse breaks down the cell membrane to form nanoscale irreversible electroporation, thereby destroying the intracellular homeostasis and resulting in necrosis or apoptosis of the left atrial appendage cells.

To achieve complete electrical isolation of the left atrial appendage, tissue ablation needs to be completely transmural, that is, both the inner and outer walls of the tissue need irreversible electroporation. Under a certain electric field intensity, the pulse ablation energy acts on the cell membrane of the target tissue, causing irreversible electroporation pores on the cells, thereby destroying the intracellular and extracellular homeostasis and achieving the effect of electrical isolation, ensuring that no electrophysiological signals can be transmitted between the left atrial appendage and the left atrium located on two sides of the target tissue, so as to treat atrial fibrillation.

High transmurality requires that the intensity of the electric field covering the inner and outer walls of the left atrial appendage must be greater than the field intensity threshold of the myocardium. Generally, the wall thickness of the left atrial appendage is 3 mm. In some embodiments, during the ablation process, the intensity of the electric field generated by the ablation element at a distance of 3 mm from its surface is greater than the field intensity threshold of the myocardium. In some embodiments, the intensity of the generated electric field is set to be greater than 400 V/cm, ensuring that the tissues of the left atrial appendages of different patients can be ablated transmurally.

The closer to the ablation element is, the denser the electric field lines are, the greater the electric field intensity is, and the easier it is to ablate the tissue. When the intensity of the electric field covering the outer wall of the left atrial appendage is above 400 V/cm, the intensity of the electric field generated on the surface of the ablation element is greater than the intensity of the electric field on the outer wall of the left atrial appendage and greater than the field intensity threshold of the myocardium of 400 V/cm.

The intensity of the electric field acting on the myocardial tissue cells is related to the pulse voltage range, the discharge area of the ablation element, and the distance between the conductive portions.

In the case where the first conductive portion is provided on the support and the second conductive portion is provided on the support or the delivery device, the pulse voltage for the ablation element (the first conductive portion and the second conductive portion) is in the range of 500 V to 4000 V, the discharge area of the first conductive portion 250 is in the range of 15 mm² to 4700 mm², and the discharge area of the second conductive portion 260 is in the range of 15 mm² to 4700 mm². In the case where the conductive portion includes a plurality of electrode elements, the discharge area refers to the sum of the discharge areas of all the electrode elements of the respective conductive portion. The distance between two closest points on the surfaces of the first conductive portion 250 and the second conductive portion 260 ranges from 1 mm to 45 mm.

Both the first conductive portion 250 and the second conductive portion 260 are provided on the frame of the left atrial appendage occlusion and ablation device 200, and the two conductive portions transmit pulse ablation electric energy with different polarities. The first conductive portion 250 and the second conductive portion 260 are provided at different axial levels of the frame of the left atrial appendage occlusion and ablation device 200 at an interval. The voltage is in the range of 500 V to 4000 V. If the voltage is too low, the intensity of the electric field generated between the two conductive portions is too low, and the ablation zone is too small for transmural ablation, resulting an unexpected ablation effect. If the voltage is too high, the left atrial appendage occlusion and ablation device 200 and the delivery device need high insulation performance, and it is difficult for the existing insulating technologies and materials to meet the withstand voltage. Moreover, if the voltage is too high, especially when the left atrial appendage occlusion and ablation device 200 and the delivery device do not have satisfied insulation performance, it is easy to cause electrical coupling between the ablation element and other parts, resulting in electric sparks and tissue eschar, and even cardiac perforation leading to pericardial effusion and other harmful results to the human body.

The two conductive portions can be in various shapes such as dot shape, rod shape, line shape or sheet shape. No matter in which shape, what matters is the distance between the two conductive portions and the discharge area of each conductive portion.

When the voltage difference between the two conductive portions remains unchanged, the distance between the two conductive portions directly affects the intensity of the electric field generated between the two electrodes. The size of the discharge area of the respective conductive portion can affect the current density around the conductive portion, further affecting the electric field.

Under a certain current, if the discharge area of the ablation element is too large, the current density will be too small, the intensity of the electric field generated on the surface of the conductive portion will be too low, the depth of the ablation zone will be shallow, and the electric field intensity at the outer wall of the left atrial appendage is difficult to reach the intensity threshold of the myocardium, so that transmural ablation is difficult to achieve, with a low success rate of ablation. If the discharge area is too small, the current density will be too high, the conductive portion is likely to be electrically coupled with other parts to generate electric sparks, and the parts of the left atrial appendage occlusion and ablation device 200 are likely to be melted or burned due to overheating, damaging the insulating material, so that the conductive portion cannot contact the wall closely.

The pulse voltage is in the range of 500 V to 4000 V, the discharge area of the ablation element is in the range of 15 mm² to 4700 mm², and the distance between the two conductive portions is in the range of 1 mm to 45 mm, which is beneficial to generate an electric field at the outer wall of the left atrial appendage with an intensity greater than the field intensity threshold of the myocardium. For example, during the ablation process, the intensity of the electric field generated by the ablation element at a distance of 3 mm from its surface is greater than 400 V/cm, which is beneficial for the ablation zone to cover the outer wall of the left atrial appendage, thereby achieving transmural ablation on the tissue of the left atrial appendage.

The above parameters can be applied to various forms of conductive portions, such as the conductive portions being at least part of the frame, or in various forms of electrodes.

Preferably, the distance between the first conductive portion 250 and the second conductive portion 260 can be further limited to the range of 3 mm to 22 mm. Within this range, the ablation zones of the two conductive portions can partially overlap with each other, that is, the first ablation zone and the second ablation zone partially overlap with each other. The two ablation zones partially overlap with each other, so that the ablation zone formed after the first ablation zone and the second ablation zone are overlapped with each other has a larger size. When the ablation zones partially overlap with each other, the ablation energy from the two conductive portions is concentrated, which can ablate the tissue with a relatively large area, facilitating transmural ablation on the tissue of the left atrial appendage with uneven thickness, and reducing the influence on the ablation effect when the frame is released at a malposition.

In this embodiment, the first conductive portion 250 and the second conductive portion 260 are spaced apart from each other in the axial direction of the frame, and the first ablation zone and the second ablation zone are formed at different axial levels of the ablation system. After the left atrial appendage occlusion and ablation device 200 is released at the ostium of the left atrial appendage, the sealing part 210 and the anchoring part 220 may have a portion that is not close to the tissue. If a conductive portion is provided at this portion, and the first ablation zone and the second ablation zone do not overlap with each other or completely overlap with each other, the ablation zone at the left atrial appendage has a relatively small range, and the chance for the ablation zone of the conductive portion to cover the outer wall of the tissue of the left atrial appendage will be reduced. In the case where the first ablation zone and the second ablation zone partially overlap with each other, the first ablation zone and the second ablation zone partially overlap with each other to form an ablation zone with a larger coverage area and a longer axial length, that is, the two ablation zones have an overlapping area and the two ablation zones are continuously distributed, so that the ablation zone with a larger coverage area formed by the two ablation zones partially overlapping with each other can cover the outer wall of the tissue of the left atrial appendage, facilitating transmural ablation with the concentrated ablation energy. The ablation zone formed by the two ablation electric fields partially overlapping with each other is convex in the circumferential direction, facilitating transmural ablation at the overlapping area of the ablation zones. It can be understood that, in the case where the first conductive portion 250 and the second conductive portion 260 are spaced apart from each other in the circumferential direction of the frame or any other directions (for example, the second conductive portion is provided on the support, the second conductive portion is provided on the delivery device, or the second conductive portion is provided independently from the support and the delivery device, and the first conductive portion and the second conductive portion are parallel or not parallel to each other), the first ablation zone and the second ablation zone partially overlapping with each other facilitate to form an ablation zone with a larger coverage area and achieve transmural ablation.

Preferably, due to the even thickness and regular shape of the ostium of the left atrial appendage, the ablation zone with a larger coverage area formed by the first ablation zone and the second ablation zone partially overlapping with each other can cover the inner wall and outer wall of the ostium of the left atrial appendage.

In some embodiments, when the pulse voltage range, the discharge area of the ablation element, and the distance between the conductive portions exceed the above-mentioned ranges, for example, the left atrial appendage occlusion and ablation device 200 can achieve transmural ablation by increasing the voltage range and reducing the distance between the two conductive portions to make the two ablation zones partially overlap with each other.

### Third Embodiment

Referring to FIG. 3, the ablation system according to this embodiment includes a left atrial appendage occlusion and ablation device 300, which is configured to occlude and ablate the left atrial appendage. The left atrial appendage occlusion and ablation device 300 includes a sealing part 310 disposed at the proximal side and an anchoring part 320 disposed at the distal side, and the sealing part 310 and the anchoring part 320 are connected to each other.

The main difference between the left atrial appendage occlusion and ablation device 300 according to this embodiment and the left atrial appendage occlusion and ablation device 200 according to the second embodiment is that the second conductive portion 360 is disposed on the proximal portion of the second frame 321.

In some embodiments, the surface of the entire second frame 321 is subjected to insulation treatment. In some embodiments, the part of the second frame 321 that is not in contact with the second conductive portion 360 is subjected to insulation treatment, while the part of the second frame 321 that is in contact with the second conductive portion 360 does not need insulation treatment.

### Fourth Embodiment

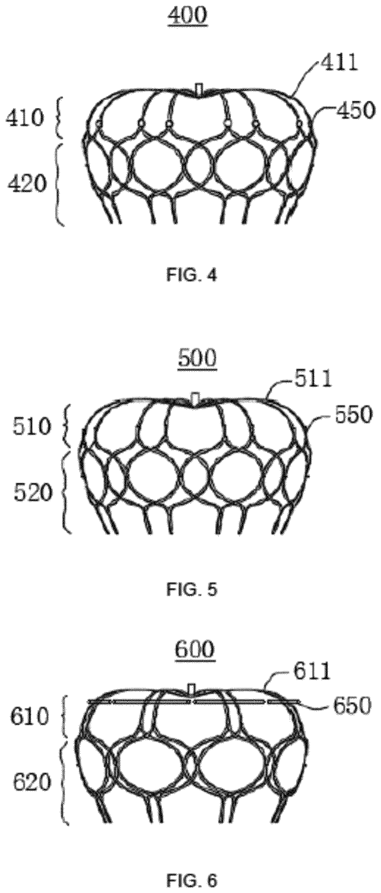

Referring to FIG. 4, the ablation system according to this embodiment includes a left atrial appendage occlusion and ablation device 400, which is configured to occlude and ablate the left atrial appendage. The left atrial appendage occlusion and ablation device 400 includes a sealing part 410 disposed at the proximal side and an anchoring part 420 disposed at the distal side, and the sealing part 410 and the anchoring part 420 are connected to each other.

The main difference between the left atrial appendage occlusion and ablation device 400 according to this embodiment and the left atrial appendage occlusion and ablation device 100 according to the first embodiment is that the first conductive portion 450 of the left atrial appendage occlusion and ablation device 400 includes a plurality of spaced electrode elements disposed on the surface of the sealing part 410. Each electrode element is a dot-shaped electrode. Each dot-shaped electrode is arranged on a different first sealing strut. In other embodiments, the number of dot-shaped electrodes on each first sealing strut can be set as required. For example, at least one first sealing strut is not provided with a dot-shaped electrode, or a plurality of dot-shaped electrodes can be disposed on the same first sealing strut.

Each dot-shaped electrode is dot-shaped. For example the dot-shaped electrode may be a granular electrode formed by coiling a straight or curved line. The section of each dot-shaped electrode is circular, elliptical, annular, rectangular, trapezoidal, or other polygonal or irregular shape.

In some embodiments, the dot-shaped electrodes of the first conductive portion 450 are configured to transmit the same ablation electric energy. In some embodiments, adjacent dot-shaped electrodes are configured to transmit ablation electric energy with different parameters, such as ablation electric energy with different polarities. In some embodiments, the ablation electric energy transmitted by each dot-shaped electrode is set as required. For example, the dot-shaped electrodes can be divided in groups, and the dot-shaped electrodes in the same group are configured to transmit ablation electric energy with the same parameters.

In some embodiments, the first conductive portion 450 is insulated from the first frame 411. Accordingly, the surface of the first frame 411 is subjected to insulation treatment, or the sides of the plurality of electrode elements of the first conductive portion 450 contacting the first frame 411 are subjected to insulation treatment.

### Fifth Embodiment

Referring to FIG. 5, the ablation system according to this embodiment includes a left atrial appendage occlusion and ablation device 500, which is configured to occlude and ablate the left atrial appendage. The left atrial appendage occlusion and ablation device 500 includes a sealing part 510 disposed at the proximal side and an anchoring part 520 disposed at the distal side, and the sealing part 510 and the anchoring part 520 are connected to each other.

The main difference between the left atrial appendage occlusion and ablation device 500 according to this embodiment and the left atrial appendage occlusion and ablation device 400 according to the fourth embodiment is that the plurality of electrode elements of the first conductive portion 550 of the left atrial appendage occlusion and ablation device 500 are all rod-shaped electrodes.

Specifically, each rod-shaped electrode is rod-shaped or strip-shaped, and the two ends of the rod-shaped electrode extend between the proximal end and the distal end. In some embodiments, two ends of the rod-shaped electrode extend in the circumferential direction of the left atrial appendage occlusion and ablation device 500. In some embodiments, the extension direction of the rod-shaped electrode is oblique relative to the axis of the left atrial appendage occlusion and ablation device 500.

In some embodiments, the rod-shaped electrodes of the first conductive portion 550 are configured to transmit the same ablation electric energy. In some embodiments, adjacent rod-shaped electrodes are configured to transmit ablation electric energy with different parameters, such as ablation electric energy with different polarities. In some embodiments, the ablation electric energy transmitted by each rod-shaped electrode is set as required. For example, the rod-shaped electrodes can be divided in groups, and the rod-shaped electrodes in the same group are configured to transmit ablation electric energy with the same parameters.

In some embodiments, the first conductive portion 550 is insulated from the first frame 511. Accordingly, the surface of the first frame 511 is subjected to insulation treatment, or the sides of the plurality of electrode elements of the first conductive portion 550 contacting the first frame 511 are subjected to insulation treatment.

### Sixth Embodiment

Referring to FIG. 6, the ablation system according to this embodiment includes a left atrial appendage occlusion and ablation device 600, which is configured to occlude and ablate the left atrial appendage. The left atrial appendage occlusion and ablation device 600 includes a sealing part 610 disposed at the proximal side and an anchoring part 620 disposed at the distal side, and the sealing part 610 and the anchoring part 620 are connected to each other.

The main difference between the left atrial appendage occlusion and ablation device 600 according to this embodiment and the left atrial appendage occlusion and ablation device 500 according to the fifth embodiment is that the plurality of electrode elements of the first conductive portions 650 of the left atrial appendage occlusion and ablation device 600 are all arc electrodes.

Specifically, each arc electrode extends in the shape of arc in the circumferential direction of the left atrial appendage occlusion and ablation device 600, and the plurality of arc electrodes are disposed on the circumferential surface of the first frame 611 at intervals.

As shown in FIG. 6, the plurality of arc electrodes defines a ring, which facilitates a ring-like ablation zone. The number of arc electrodes can be set as required.

The circumferential angles of different arc electrodes in the circumferential direction of the left atrial appendage occlusion and ablation device 600 do not overlap each other. In some embodiments, the circumferential angles of different arc electrodes in the circumferential direction of the left atrial appendage occlusion and ablation device 600 at least partially overlap each other.

As shown in FIG. 6, the arc electrodes are located at the same axial level of the left atrial appendage occlusion and ablation device 600. In some other embodiments, adjacent arc electrodes are located at different axial levels.

In some embodiments, the arc electrodes of the first conductive portion 650 are configured to transmit the same ablation electric energy. In some embodiments, adjacent arc electrodes are configured to transmit ablation electric energy with different parameters, such as ablation electric energy with different polarities. In some embodiments, the ablation electric energy transmitted by each arc electrode is set as required. For example, the arc electrodes can be divided in groups, and the arc electrodes in the same group are configured to transmit ablation electric energy with the same parameters.

In some embodiments, the first conductive portion 650 is insulated from the first frame 611. Accordingly, the surface of the first frame 611 is subjected to insulation treatment, or the sides of the plurality of electrode elements of the first conductive portion 650 contacting the first frame 611 are subjected to insulation treatment.

### Seventh Embodiment

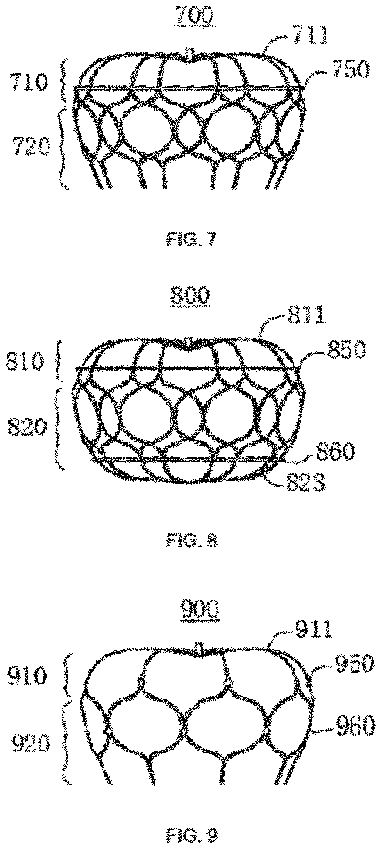

Referring to FIG. 7, the ablation system according to this embodiment includes a left atrial appendage occlusion and ablation device 700, which is configured to occlude and ablate the left atrial appendage. The left atrial appendage occlusion and ablation device 700 includes a sealing part 710 disposed at the proximal side and an anchoring part 720 disposed at the distal side, and the sealing part 710 and the anchoring part 720 are connected to each other.

The main difference between the left atrial appendage occlusion and ablation device 700 according to this embodiment and the left atrial appendage occlusion and ablation device 600 according to the sixth embodiment is that the first conductive portion 750 of the left atrial appendage occlusion and ablation device 700 includes one electrode element in the form of a ring electrode. For the detail of the ring electrode, please refer to the above-mentioned embodiment.

A second conductive portion that cooperates with the first conductive portion 750 can be provided in the left atrial appendage occlusion and ablation device 700, or the second conductive portion can be provided in the delivery device, or the second conductive portion can be provided independently from the left atrial appendage occlusion and ablation device 700 and the delivery device. The detail please refers to the first embodiment.

### Eighth Embodiment

Referring to FIG. 8, the ablation system according to this embodiment includes a left atrial appendage occlusion and ablation device 800, which is configured to occlude and ablate the left atrial appendage. The left atrial appendage occlusion and ablation device 800 includes a sealing part 810 disposed at the proximal side and an anchoring part 820 disposed at the distal side, and the sealing part 810 and the anchoring part 820 are connected to each other.

The main difference between the left atrial appendage occlusion and ablation device 800 according to this embodiment and the left atrial appendage occlusion and ablation device 700 according to the seventh embodiment is that the distal end of the anchoring part 820 of the left atrial appendage occlusion and ablation device 800 is closed, and the second conductive portion 860 is disposed on the frame of the left atrial appendage occlusion and ablation device 800, specifically on the anchoring part 820. The second conductive portion 860 is an electrode element disposed on the surface of the anchoring part 820 in the form of a ring electrode.

In this embodiment, the distal end of the anchoring part 820 is closed, that is, the distal ends of the second anchoring struts 823 are joined together to close the distal end of the anchoring part 820. In some embodiments, the distal end of the anchoring part 820 is further provided with a connecting piece for connecting the ends of the second anchoring struts 823.

The position of the second conductive portion 860 disposed on the anchoring part 820 can be set as required.

The electric field generated between the first conductive portion 850 and the second conductive portion 860 is configured to ablate the tissue at the ostium of the left atrial appendage. The detail for the ring electrodes of the first conductive portion 850 and the second conductive portion 860 refer to the above-mentioned embodiment.

### Ninth Embodiment

Referring to FIG. 9, the ablation system according to this embodiment includes a left atrial appendage occlusion and ablation device 900, which is configured to occlude and ablate the left atrial appendage. The left atrial appendage occlusion and ablation device 900 includes a sealing part 910 disposed at the proximal side and an anchoring part 920 disposed at the distal side, and the sealing part 910 and the anchoring part 920 are connected to each other.

The main difference between the left atrial appendage occlusion and ablation device 900 according to this embodiment and the left atrial appendage occlusion and ablation device 800 according to the eighth embodiment is that the first conductive portion 950 and the second conductive portion 960 disposed on the frame of the left atrial appendage occlusion and ablation device 900 include dot-shaped electrodes, and the detail for the dot-shaped electrode refers to the above-mentioned embodiment.

### Tenth Embodiment

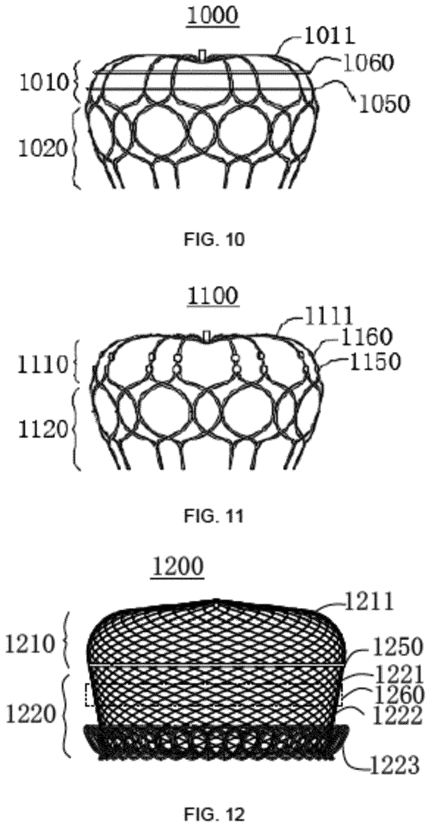

Referring to FIG. 10, the ablation system according to this embodiment includes a left atrial appendage occlusion and ablation device 1000, which is configured to occlude and ablate the left atrial appendage. The left atrial appendage occlusion and ablation device 1000 includes a sealing part 1010 disposed at the proximal side and an anchoring part 1020 disposed at the distal side, and the sealing part 1010 and the anchoring part 1020 are connected to each other.

The main difference between the left atrial appendage occlusion and ablation device 1000 according to this embodiment and the left atrial appendage occlusion and ablation device 800 according to the eighth embodiment is that both the first conductive portion 1050 and the second conductive portion 1060 in the left atrial appendage occlusion and ablation device 1000 are disposed on the sealing part 1010.

The first conductive portion 1050 and the second conductive portion 1060 are located at different axial levels of the sealing part 1010, and the first conductive portion 1050 is disposed on the distal side of the second conductive portion 1060. Both the first conductive portion 1050 and the second conductive portion 1060 are in the form of a ring electrode. The detail for the ring electrode refers to the above-mentioned embodiment.

Since the first conductive portion 1050 is disposed on the edge area of the sealing part 1010, preferably, the first conductive portion 1050 is disposed at the portion of the sealing part 1010 with the maximum radial dimension. Accordingly, the second conductive portion 1060 can be arranged at the proximal side or the distal side of the first conductive portion 1050 as required.

### Eleventh Embodiment

Referring to FIG. 11, the ablation system according to this embodiment includes a left atrial appendage occlusion and ablation device 1100, which is configured to occlude and ablate the left atrial appendage. The left atrial appendage occlusion and ablation device 1100 includes a sealing part 1110 disposed at the proximal side and an anchoring part 1120 disposed at the distal side, and the sealing part 1110 and the anchoring part 1120 are connected to each other.

The main difference between the left atrial appendage occlusion and ablation device 1100 according to this embodiment and the left atrial appendage occlusion and ablation device 1000 according to the tenth embodiment is that the first conductive portion 1150 and the second conductive portion 1160 provided on the frame of the left atrial appendage occlusion and ablation device 1100 include dot-shaped electrodes, and the detail for the dot-shaped electrode refers to the above-mentioned embodiment.

### Twelfth Embodiment

Referring to FIG. 12, the ablation system according to this embodiment includes a left atrial appendage occlusion and ablation device 1200, which is configured to occlude and ablate the left atrial appendage. The left atrial appendage occlusion and ablation device 1200 includes a sealing part 1210 disposed at the proximal side and an anchoring part 1220 disposed at the distal side, and the sealing part 1210 and the anchoring part 1220 are connected to each other.

The main difference between the left atrial appendage occlusion and ablation device 1200 according to this embodiment and the left atrial appendage occlusion and ablation device 700 according to the seventh embodiment is that the sealing part 1210 and the anchoring part 1220 of the left atrial appendage occlusion and ablation device 1200 have a different structure from the seventh embodiment.

Specifically, the left atrial appendage occlusion and ablation device 1200 is made by weaving. The sealing part 1210 is the proximal portion of the left atrial appendage occlusion and ablation device 1200, and the anchoring part 1220 is the distal portion of the left atrial appendage occlusion and ablation device 1200. The anchoring part 1220 includes a main body 1222 and a reinforcing ring 1223 disposed at the distal end of the main body 1222 and extending in the circumferential direction of the main body.

The reinforcing ring 1223 is fixed on the outer peripheral surface of the main body 1222. The part of the reinforcing ring 1223 close to the main body 1222 is connected with the main body 1222. The part of the reinforcing ring 1223 away from the main body 1222 in the radial direction protrudes away from the axis of the left atrial appendage occlusion and ablation device 1200 in the radial direction, that is, the reinforcing ring 1223 protrudes from the outer periphery of the distal end of the main body 1222. The reinforcing ring 1223 is configured to be released inside the left atrial appendage, contact and abut against the tissue of the left atrial appendage, to improve the anchoring stability of the left atrial appendage occlusion and ablation device 1200.

The reinforcing ring 1223 includes a plurality of ring-shaped support coils connected to each other in the circumferential direction, and the plurality of ring-shaped support coils are arranged in a circle in the circumferential direction. In some embodiments, each ring-shaped support coil is shaped as a complete ring. In some embodiments, each ring-shaped support coil has a curved structure and is not shaped as a complete ring, and the ends of adjacent ring-shaped support coils are connected to form the reinforcing ring 1223.

The sealing part 1210 and the main body 1222 can be formed in one piece by weaving. In some embodiments, the left atrial appendage occlusion and ablation device 1200 is formed in one piece by weaving.

In this embodiment, the first conductive portion 1250 is an electrode element disposed on the surface of the sealing part 1210 in the form of a ring electrode. For the detail of the ring electrode, please refer to the above-mentioned embodiment.

As shown in FIG. 12, in some embodiments, the second conductive portion 1260 is provided on the left atrial appendage occlusion and ablation device 1200. The second conductive portion 1260 is at least part of the second frame 1221. In some embodiments, the second conductive portion 1260 is at least part of the frame of the reinforcing ring 123. The first conductive portion 1250 interacts with the second conductive portion 1260 to form a ring-like ablation zone at the ostium of the left atrial appendage. For alternatives of the second conductive portion, please refer to the seventh and first embodiments.

In some embodiments, the reinforcing ring 1223 may be disposed on the sealing part 1210, protruding from the sealing part 1210 in a circumferential direction, and configured to occlude the ostium of the left atrial appendage. In such case, the first conductive portion 1250 can be at least part of the frame of the reinforcing ring 1223, so as to form a ring-like ablation zone at the ostium of the left atrial appendage.

### Thirteenth Embodiment

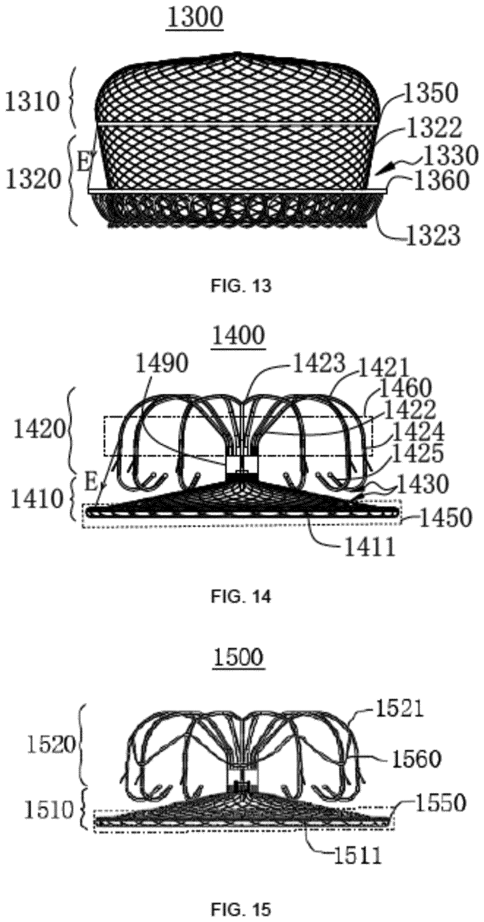

Referring to FIG. 13, the ablation system according to this embodiment includes a left atrial appendage occlusion and ablation device 1300, which is configured to occlude and ablate the left atrial appendage. The left atrial appendage occlusion and ablation device 1300 includes a sealing part 1310 disposed at the proximal side and an anchoring part 1320 disposed at the distal side, and the sealing part 1310 and the anchoring part 1320 are connected to each other.

The main difference between the left atrial appendage occlusion and ablation device 1300 according to this embodiment and the left atrial appendage occlusion and ablation device 1200 according to the twelfth embodiment is that the second conductive portion 1360 is an electrode element disposed on the surface of the anchoring part 1320 in the form of a ring electrode. For the detail of the ring electrode, please refer to the above-mentioned embodiment.

One radial end of the reinforcing ring 1323 is connected to the main body 1322, and the other radial end of the reinforcing ring 1323 is a free end protruding radially and outwardly relative to the main body. The second conductive portion 1360 is disposed on the free end of the reinforcing ring 1323. In some embodiments, the second conductive portion 1360 is disposed at the portion of the reinforcing ring with the maximum radial dimension, so that the second conductive portion 1360 can contact the tissue. The centers of the ring-shaped support coils of the reinforcing ring 1323 are communicated to form a channel, and in some embodiments, the second conductive portion 1360 passes through the channel formed by the reinforcing ring 1323.

The left atrial appendage occlusion and ablation device 1300 includes an accommodating part 1330. The accommodating part 1330 is adjacent to the axis of the support relative to the first conductive portion 1350 and/or the second conductive portion 1360. In this embodiment, the accommodating part 1330 is adjacent to the axis of the support relative to the first conductive portion 1350 and the second conductive portion 1360, so that an accommodating space is defined outside the outer peripheral wall of the accommodating part 1330 for receiving the tissue of the left atrial appendage.

The shortest line between any point on the first conductive portion 1350 and any point on the second conductive portion 1360 is a shortest electric field line E. The electric field near the shortest electric field line E has denser electric field lines than the other positions in the electric field, and higher electric field intensity which is more likely to be greater than the field intensity threshold of the target tissue, so the tissue where the shortest electric field line E passes through is easy to be ablated, and the ablation effect is better.

The first conductive portion 1350 and the second conductive portion 1360 are arranged on opposite sides of the accommodating space. During the ablation process, at least one shortest electric field line passes through the area outside the support, i.e., the area away from the axis of the frame, specifically, the accommodating space defined outside the accommodating part 1330 of the frame, which facilitates the shortest electric field line E to pass through the tissue located in the accommodating space. Preferably, at least one shortest electric field line E passing through the accommodating space is inclined relative to the axis of the left atrial appendage occlusion and ablation device 1300, so that the accommodating part 1330 can accommodate more tissue of the left atrial appendage, and can reduce the difficulty for the shortest electric field line E to pass through the tissue in the accommodating space.

Specifically, as shown in FIG. 13, the radial dimension of the main body 1322 between the first conductive portion 1350 and the second conductive portion 1360 for contacting the tissue wall of the left atrial appendage gradually decreases from the proximal end to the distal end (the direction from the sealing part to the anchoring part), the second conductive portion 1360 is disposed at the portion of the reinforcing ring 1323 with the maximum radial dimension, and the radial dimension of the second conductive portion 1360 is greater than the radial dimension of the first conductive portion 1350. Accordingly, the part of the frame of the left atrial appendage occlusion and ablation device 1300 between the first conductive portion 1350 and the second conductive portion 1360 is the accommodating part 1330, and the accommodating space is defined outside the outer wall of the accommodating part 1330 for accommodating the tissue of the left atrial appendage. When the left atrial appendage occlusion and ablation device 1300 is implanted into the left atrial appendage, the outer wall of the accommodating part 1330 is at least partially configured to contact the tissue of the left atrial appendage.

The left atrial appendage occlusion and ablation device 1300 is made by weaving. The accommodating part 1330 between the first conductive portion 1350 and the second conductive portion 1360 extends in the circumferential direction of the left atrial appendage occlusion and ablation device 1300. The first conductive portion 1350 and the second conductive portion 1360 are spaced apart from each other in the axial direction, so that the tissue of the left atrial appendage in a circle can be accommodated to the accommodating part 1330. The first conductive portion 1350 and the second conductive portion 1360 perform ablation on the tissue in the accommodating part 1330, forming a continuous ring-like ablation zone in the left atrial appendage.

At least one shortest electric field line E passing through the accommodating space is inclined relative to the axis of the left atrial appendage occlusion and ablation device 1300. The radial dimension of the second conductive portion 1360 is greater than the radial dimension of the first conductive portion 1350, so that at least one shortest electric field line E passing through the accommodating space is inclined relative to the axis of the left atrial appendage occlusion and ablation device 1300, so as to facilitate the shortest electric field line E to pass through the tissue in the accommodating space.

It can be understood that the accommodating part 1330 is not limited to be the frame of the left atrial appendage occlusion and ablation device according to the embodiments of the present disclosure, but may be other structure between the supporter of the first conductive portion and the supporter of the second conductive portion that is recessed toward the axis of the left atrial appendage occlusion and ablation device, so that the above-mentioned accommodating space can be defined between the supporter of the first conductive portion and the supporter of the second conductive portion. That is, the accommodating part for accommodating the target tissue can be arranged on the support and delivery device.

### Fourteenth Embodiment

Referring to FIG. 14, the ablation system according to this embodiment includes a left atrial appendage occlusion and ablation device 1400, which is configured to occlude and ablate the left atrial appendage. The left atrial appendage occlusion and ablation device 1400 includes a sealing part 1410 disposed at the proximal side and an anchoring part 1420 disposed at the distal side, and the sealing part 1410 and the anchoring part 1420 are connected to each other.

The main difference between the left atrial appendage occlusion and ablation device 1400 according to this embodiment and the left atrial appendage occlusion and ablation device 100 according to the first embodiment is that the left atrial appendage occlusion and ablation device 1400 has a double-disc structure. The sealing part 1410 is disc-shaped, the anchoring part 1420 is disc-shaped, and the sealing part 1410 is insulated from and connected with the anchoring part 1420 through a connecting piece 1490. At least part of the connecting piece 1490 can be made of insulating material, thereby providing an insulation connection between the sealing part 1410 and the anchoring part 1420.

Specifically, the first frame 1411 of the sealing part 1410 is made by weaving. Seen from the perspective of FIG. 14, the distal surface of the sealing part 1410 is conical, and it can be understood that the proximal surface of the sealing part 1410 can be planar or conical or in other shapes.

The second frame 1421 of the anchoring part 1420 is made by laser cutting a tube. The second frame 1421 includes first anchoring struts 1422, second anchoring struts 1423, third anchoring struts 1424, and fourth anchoring struts 1425 connected in sequence.

The proximal end of the first anchoring strut 1422 is connected to the connecting piece 1490, and the first anchoring strut 1422 extends between the proximal end and the distal end. One end of each second anchoring strut 1423 is connected to a corresponding first anchoring strut 1422, the other ends of the adjacent two second anchoring struts 1423 are joined together and connected with the corresponding third anchoring strut 1424. The third anchoring strut 1424 extends between the proximal end and the distal end and outside the first anchoring strut 1422 for contacting the inner wall of the left atrial appendage. One end of the third anchoring strut 1424 is connected to the fourth anchoring strut 1425. One end of the fourth anchoring strut 1425 is connected to the third anchoring strut 1424, and the other end of the fourth anchoring strut 1425 is a free end which extends distally toward the axis.

The first conductive portion 1450 is provided on the sealing part 1410. The first conductive portion 1450 is at least part of the first frame 1411 of the sealing part 1410, such as part of the first frame 1411 or the entire first frame 1411. As shown in FIG. 14, the first conductive portion 1450 is the peripheral edge area of the sealing part 1410, and is provided at least at the portion of the peripheral edge area of the sealing part 1410 with the maximum radial dimension. Further, the first conductive portion 1450 is provided at least on the peripheral edge area of the sealing part 1410 facing the anchoring part 1420, for contacting the ostium of the left atrial appendage.

Optionally, the anchoring part 1420 can use at least part of the second frame 1421 as the second conductive portion 1460, and the pulse ablation electric field generated between the first conductive portion 1450 and the second conductive portion 1460 is configured to ablate the tissue.

In the pulse ablation electric field, the electric field line is directed from the first conductive portion 1450 to the second conductive portion 1460, or from the second conductive portion 1460 to the first conductive portion 1450. Along the direction of the electric field line, the closer to the first conductive portion 1450 or the second conductive portion 1460 is, the denser the electric field lines are, the high the ablation electric field intensity is, and the easier it is to achieve transmural ablation zone.

Since the tissue at the ostium of the left atrial appendage contacts the first conductive portion 1450, and the electric field lines point to the second conductive portion 1460 provided on the anchoring part 1420, or point from the second conductive portion 1460 to the first conductive portion 1450, most of the electric field lines will pass through the tissue at the ostium of the left atrial appendage. The electric field lines through the tissue at the ostium of the left atrial appendage are denser, and the electric field intensity is higher, so it is easy to form a transmural ablation zone with an electric field intensity greater than the field intensity threshold for myocardial injury and passing through the tissue at the ostium of the left atrial appendage, and the ablation success rate is relatively high.

In another aspect, the shortest line between any point on the first conductive portion 1450 and any point on the second conductive portion 1460 is the shortest electric field line E between the first conductive portion 1450 and the second conductive portion 1460. The position in the electric field near the shortest electric field line E has a higher electric field intensity than other positions in the electric field, with a concentrated electric field energy.

FIG. 14 is a view of the frame in a natural expanded state without external force. The radial dimension of the sealing part 1410 is greater than that of the ostium of the left atrial appendage, so that the edge area of the sealing part 1410 facing the anchoring part 1420 with a larger radial dimension can contact the tissue at the ostium of the left atrial appendage close to or facing the left atrium, where the tissue at the ostium of the left atrial appendage is sandwiched between the sealing part 1410 and the anchoring part 1420.

As shown in FIG. 14, the distal surface of the sealing part 1410 is conical, and the radial dimension gradually decreases from the proximal end to the distal end. The first conductive portion 1450 is at least part of the first frame 1411. The frame of the left atrial appendage occlusion and ablation device 1400 includes an accommodating part 1430 between the first conductive portion 1450 and the second conductive portion 1460. Specifically, the accommodating part 1430 includes the portions of the first frame and the second frame facing and close to each other. An accommodating space is defined outside the outer wall of the accommodating part 1430 for accommodating the tissue of the left atrial appendage. When the left atrial appendage occlusion and ablation device 1400 is implanted into the left atrial appendage, the outer wall of the accommodating part 1430 is at least partially configured to contact the tissue of the left atrial appendage. Moreover, the radial dimension of the sealing part 1410 is greater than that of the anchoring part 1420, and the shortest electric field line E is inclined at a certain angle relative to the axis of the left atrial appendage occlusion and ablation device 1400, so that the shortest electric field line can pass through the deep tissue at the ostium of the left atrial appendage, and an electric field with an intensity greater than the electric field threshold of the myocardium can be generated within a certain thickness range of the ostium of the left atrial appendage, thereby achieving transmural ablation at the ostium of the left atrial appendage.

In the first embodiment shown in FIG. 1, if the second conductive portion is disposed at the distal side of the sealing part 110, such as on the anchoring part 120 or the delivery device, or independent from the left atrial appendage occlusion and ablation device 100 and the delivery device, the tissue of the left atrial appendage near the first conductive portion 120 and the second conductive portion, especially the tissue contacting the first conductive portion 120 and the second conductive portion has a better ablation effect. When the shortest electric field line passes through the surface of the tissue of the left atrial appendage, or passes through the deep tissue of the left atrial appendage, transmural ablation on the tissue with a relatively large thickness can be achieved. Because the thickness of the tissue at the ostium of the left atrial appendage is relatively uniform and the shape is regular, it is convenient to perform ablation there, and the difficulty of transmural ablation is relatively low.

If radio frequency ablation is applied, the tissue contacting the first conductive portion 1450 is relatively easy to be ablated.

The above technical effects can be achieved by other embodiments, that is, the above technical effects can be achieved by using relevant technical features in other embodiments, and will not be repeated in other embodiments.

As shown in FIG. 14, in some embodiments, an electrode element can be additionally disposed on the second frame 1421 as the second conductive portion 1460. Alternatively, the second conductive portion can be provided outside the left atrial appendage occlusion and ablation device 1400, for example, on the delivery device for the left atrial appendage occlusion and ablation device 1400, or the second conductive portion can be provided independently from the left atrial appendage occlusion and ablation device 1400 and the delivery device.

As shown in FIG. 14, the first conductive portion 1450 is provided on the proximal side of the frame, and the second conductive portion 1460 is provided at the distal side of the first conductive portion 1450. The radial dimensions of the portions of the frame where the two conductive portions are provided can be different or the same. As shown in FIG. 14, the radial dimension of the first conductive portion 1450 is greater than the radial dimension of the second conductive portion 1460. In some embodiments, the first conductive portion 1450 is provided on the peripheral edge area of the first frame 1411 with the maximum radial dimension. For example, a ring electrode can be disposed on the peripheral edge area of the proximal end of the first frame 1411 with the maximum radial dimension. Since the radial dimension of the first conductive portion 1450 is greater than the radial dimension of the second conductive portion 1460, it is convenient for the shortest electric field line E to pass through the tissue at the ostium. As shown in FIG. 14, the first conductive portion 1450 is provided at the proximal end of the sealing part 1410, and the first conductive portion 1450 is part of the first frame 1411 at the proximal end. The shortest electric field line E is the shortest line between any point on the first conductive portion 1450 and any point on the second conductive portion 1460. The shortest electric field line E passes through the area outside the frame and thus can pass through the tissue.

When the left atrial appendage occlusion and ablation device 1400 is delivered to the ostium of the left atrial appendage, the shortest electric field line E between the first conductive portion 1450 and the second conductive portion 1460 can easily pass through the area outside the frame, and the frame between the two conductive portions is used to contact the tissue, so the shortest electric field line can easily pass through the tissue during the ablation process.

### Fifteenth Embodiment

Referring to FIG. 15, the ablation system according to this embodiment includes a left atrial appendage occlusion and ablation device 1500, which is configured to occlude and ablate the left atrial appendage. The left atrial appendage occlusion and ablation device 1500 includes a sealing part 1510 disposed at the proximal side and an anchoring part 1520 disposed at the distal side, and the sealing part 1510 and the anchoring part 1520 are connected to each other.

The main difference between the left atrial appendage occlusion and ablation device 1500 according to this embodiment and the left atrial appendage occlusion and ablation device 1400 according to the fourteenth embodiment is that the second conductive portion 1560 is disposed on the left atrial appendage occlusion and ablation device 1500. The second conductive portion 1560 is an electrode element disposed on the surface of the anchoring part 1520. Specifically, the electrode element is a wave-like electrode.

Specifically, the wave-like electrode extends in the circumferential direction and the axial direction to form a preset electrode pattern. For example, as shown in FIG. 15, the wave-like electrode extends in the circumferential direction of the left atrial appendage occlusion and ablation device 1500 and also in the axial direction alternatively toward the proximal end or the distal end so as to form a wave-like pattern in the circumferential direction on the side wall of the anchoring part 1520, increasing the area of the second conductive portion 1560 in the axial direction and thus the ablation range, and facilitating the load and release of the left atrial appendage occlusion and ablation device 1500.

In this embodiment, the first conductive portion 1550 and the second conductive portion 1560 meet the parameters set in the previous embodiments. For example, during the ablation, the intensity of the electric field generated by the first conductive portion 1550 and the second conductive portion 1560 at a distance of 3 mm from the respective surfaces thereof is greater than 400 V/cm. In some embodiments, the left atrial appendage occlusion and ablation device 1500 is configured to ablate cardiac tissue other than the left atrial appendage 10, for example, the pulmonary vein which generally has a thickness of 2 mm. During the ablation process, the intensity of the electric field generated by the ablation element at a distance of 2 mm from its surface is greater than the field intensity threshold of the myocardium, for example, is greater than 400 V/cm, thereby achieving transmural ablation.

In the case where the ablation element is provided on the support and/or the delivery device, the pulse voltage for the ablation element (the first conductive portion 1550 and the second conductive portion 1560) is in the range of 500 V to 4000 V, the discharge area of the first conductive portion 1550 is in the range of 15 mm² to 4700 mm², and the discharge area of the second conductive portion 1560 is in the range of 15 mm² to 4700 mm². In the case where the conductive portion includes a plurality of electrode elements, the discharge area refers to the sum of the discharge areas of all the electrode elements or the frame of the conductive portion. The distance between two closest points on the surfaces of the first conductive portion 1550 and the second conductive portion 1560 ranges from 1 mm to 45 mm. Preferably, the distance between the first conductive portion 1550 and the second conductive portion 1560 can be further limited to the range of 3 mm to 22 mm. Within this range, the ablation zones of the two conductive portions can partially overlap with each other.

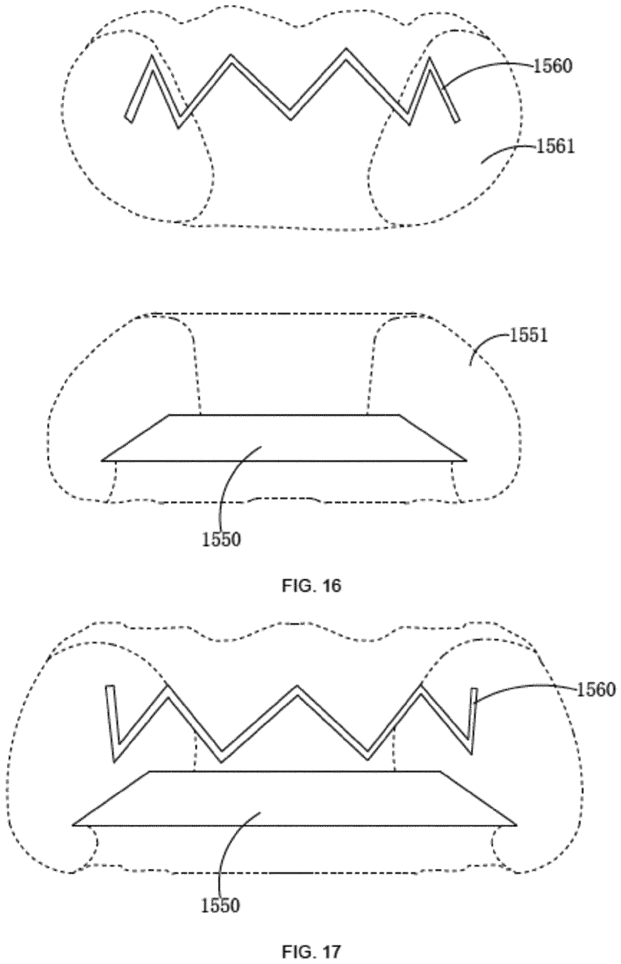

FIG. 16 is a schematic view showing the electric field in which the ablation zones of the ablation element in an embodiment are spaced apart from each other, and FIG. 17 is a schematic view showing the electric field in which the ablation zones of the ablation element in an embodiment are overlapped with each other. In FIG. 16, the zone where the intensity of the electric field generated around the first conductive portion 1550 is greater than the intensity threshold of the myocardium is the first ablation zone 1551, and the zone where the intensity of the electric field generated around the second conductive portion 1560 is greater than the intensity threshold of the myocardium is the second ablation zone 1561. FIG. 16 and FIG. 17 are schematic views showing the distribution of the ablation zones with electric field intensity greater than or equal to 400 V/cm when the pulse frequency ranges from 500 Hz to 3 MHz. The ablation element in FIG. 17 is the ablation element in the fifteenth embodiment shown in FIG. 15. The ablation element includes a first conductive portion 1550 and a second conductive portion 1560. The first conductive portion 1550 is at least part of the frame of the sealing part 1510. The second conductive portion 1560 is a wave-like electrode element disposed on the anchoring part 1520. It can be seen from FIG. 16 that the ablation zones of the two conductive portions do not overlap each other, but respectively form an isosurface. When the left atrial appendage occlusion and ablation device corresponding to FIG. 16 occludes and ablates the ostium of the left atrial appendage, the ablation zones formed by the two conductive portions do not overlap with each other, and the intensity of the electric field in the space between the two ablation zones is too low so that a complete electrical isolation cannot be achieved. In the axial direction, the size of the first ablation zone 1551 is smaller than the size of the ablation zone formed after the two ablation zones partially overlap with each other as shown in FIG. 17 (i.e., the area defined by the outer outline of the dotted line in FIG. 17). When the left atrial appendage occlusion and ablation device is implanted into the ostium of the left atrial appendage, the sealing part may be deflected at a certain angle relative to the ostium, that is, the sealing part may contact the ostium of the left atrial appendage loosely at some positions, and the first ablation zone 1551 may cover the tissue inside the ostium of the left atrial appendage (between the inner wall and the outer wall), but cannot completely cover the outer wall of the ostium of the left atrial appendage, so it is difficult to achieve transmural ablation at the ostium, with unexpected ablation effect. However, if the ablation element is arranged in such a manner that the ablation zones partially overlap with each other as shown in FIG. 17, the axial dimension of the overlapping ablation zone is greater than that of any single ablation zone, so that the energy of the two ablation zones can be concentrated to ablate one site, improving the ablation success rate of the area to be ablated. That is to say, after the device is implanted into the ostium of the left atrial appendage, even the peripheral edge area of the sealing part 1510 contacts the ostium loosely at some positions, since the ablation element has a relatively large ablation zone in the axial direction, it is easier to achieve transmural ablation around the sealing part 1510 than two spaced ablation zones with smaller axial dimensions.

The ablation zones partially overlapping with each other include proximal and distal parts in the axial direction. Even if the proximal part cannot contact the wall closely and its peripheral edge is difficult to cover the outer wall of the left atrial appendage, the distal part can cover the outer wall of the left atrial appendage, thereby reducing the influence of the released sealing part contacting the wall loosely on the ablation effect, and improving the success rate of ablation.

In this embodiment, the first conductive portion 1550 is provided on the sealing part 1510, and the ablation zone formed by the first ablation zone and the second ablation zone partially overlapping with each other is distributed at the ostium of the left atrial appendage, so as to ablate the ostium of the left atrial appendage. Compared with the inner tissue of the left atrial appendage, the tissue at the ostium of the left atrial appendage has a smooth surface and a regular shape, so that the first conductive portion 1550 is more likely to contact the wall, and the tissue at the ostium of the left atrial appendage has a relatively even thickness, so that the ablation zone can achieve continuous transmural ablation at the ostium.

Since the wall thickness of the left atrial appendage is generally 3 mm, and the distance between the boundary of the two overlapping ablation zones and the surfaces of the conductive portions is larger than the wall thickness of the left atrial appendage, so transmural ablation can be achieved.

### Sixteenth Embodiment

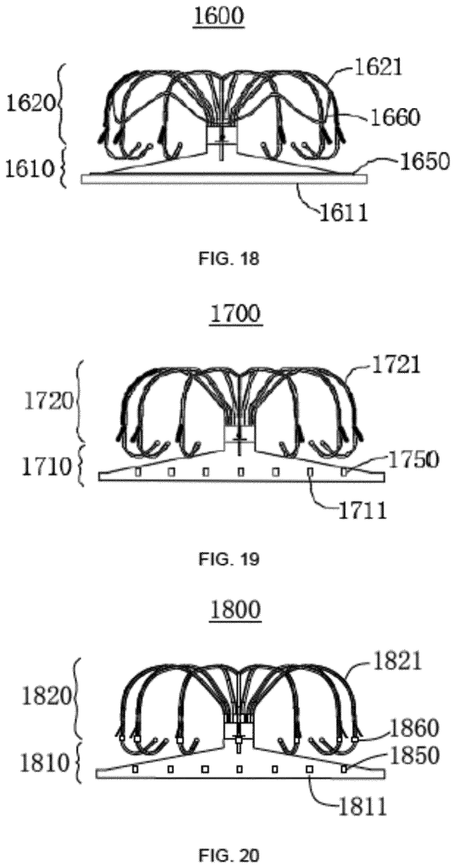

Referring to FIG. 18, the ablation system according to this embodiment includes a left atrial appendage occlusion and ablation device 1600, which is configured to occlude and ablate the left atrial appendage. The left atrial appendage occlusion and ablation device 1600 includes a sealing part 1610 disposed at the proximal side and an anchoring part 1620 disposed at the distal side, and the sealing part 1610 and the anchoring part 1620 are connected to each other.

The main difference between the left atrial appendage occlusion and ablation device 1600 according to this embodiment and the left atrial appendage occlusion and ablation device 1500 according to the fifteenth embodiment is that the first conductive portion 1650 is an electrode element disposed on the surface of the sealing part 1610 in the form of a ring electrode. For the detail of the ring electrode, please refer to the above-mentioned embodiment.

### Seventeenth Embodiment

Referring to FIG. 19, the ablation system according to this embodiment includes a left atrial appendage occlusion and ablation device 1700, which is configured to occlude and ablate the left atrial appendage. The left atrial appendage occlusion and ablation device 1700 includes a sealing part 1710 disposed at the proximal side and an anchoring part 1720 disposed at the distal side, and the sealing part 1710 and the anchoring part 1720 are connected to each other.

The main difference between the left atrial appendage occlusion and ablation device 1700 according to this embodiment and the left atrial appendage occlusion and ablation device 1400 according to the fourteenth embodiment is that the first conductive portion 1750 is an electrode element provided on the surface of the sealing part 1710. Specifically, the electrode element includes a plurality of dot-shaped electrodes arranged at intervals. For the detail of the dot-shaped electrode, please refer to the above-mentioned embodiment. The second conductive portion is not provided on the frame, but provided on a component other than the frame.

### Eighteenth Embodiment

Referring to FIG. 20, the ablation system according to this embodiment includes a left atrial appendage occlusion and ablation device 1800, which is configured to occlude and ablate the left atrial appendage. The left atrial appendage occlusion and ablation device 1800 includes a sealing part 1810 disposed at the proximal side and an anchoring part 1820 disposed at the distal side, and the sealing part 1810 and the anchoring part 1820 are connected to each other.

The main difference between the left atrial appendage occlusion and ablation device 1800 according to this embodiment and the left atrial appendage occlusion and ablation device 1700 according to the seventeenth embodiment is that the second conductive portion 1860 is disposed on the left atrial appendage occlusion and ablation device 1800. Specifically, the second conductive portion 1860 is disposed on the anchoring part 1820, and includes a plurality of dot-shaped electrodes arranged at intervals on the surface of the anchoring part 1820. For the detail of the dot-shaped electrode, please refer to the above-mentioned embodiment.

### Nineteenth Embodiment

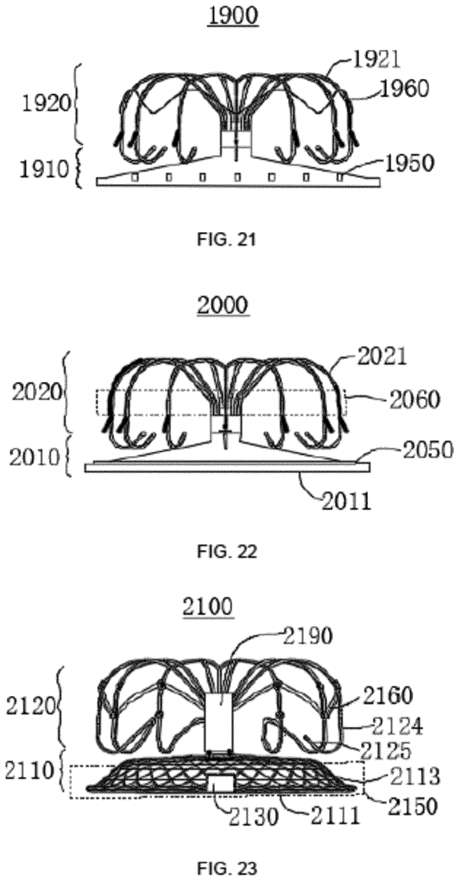

Referring to FIG. 21, the ablation system according to this embodiment includes a left atrial appendage occlusion and ablation device 1900, which is configured to occlude and ablate the left atrial appendage. The left atrial appendage occlusion and ablation device 1900 includes a sealing part 1910 disposed at the proximal side and an anchoring part 1920 disposed at the distal side, and the sealing part 1910 and the anchoring part 1920 are connected to each other.

The main difference between the left atrial appendage occlusion and ablation device 1900 according to this embodiment and the left atrial appendage occlusion and ablation device 1800 according to the eighteenth embodiment is that the second conductive portion 1960 provided on the surface of the anchoring part 1920 is a wave-like electrode. For the detail of the wave-like electrode, please refer to the above-mentioned embodiment.

### Twentieth Embodiment

Referring to FIG. 22, the ablation system according to this embodiment includes a left atrial appendage occlusion and ablation device 2000, which is configured to occlude and ablate the left atrial appendage. The left atrial appendage occlusion and ablation device 2000 includes a sealing part 2010 disposed at the proximal side and an anchoring part 2020 disposed at the distal side, and the sealing part 2010 and the anchoring part 2020 are connected to each other.

The main difference between the left atrial appendage occlusion and ablation device 2000 according to this embodiment and the left atrial appendage occlusion and ablation device 1600 according to the sixteenth embodiment is that the second conductive portion 2060 is part of the second frame 2021 of the anchoring part 2020.

### Twenty-first embodiment

Referring to FIG. 23, the ablation system according to this embodiment includes a left atrial appendage occlusion and ablation device 2100, which is configured to occlude and ablate the left atrial appendage. The left atrial appendage occlusion and ablation device 2100 includes a sealing part 2110 disposed at the proximal side and an anchoring part 2120 disposed at the distal side, and the sealing part 2110 and the anchoring part 2120 are connected to each other.

The main difference between the left atrial appendage occlusion and ablation device 2100 according to this embodiment and the left atrial appendage occlusion and ablation device 1500 according to the fifteenth embodiment is the specific structures of the sealing part 2110 and the anchoring part 2120.

Specifically, in this embodiment, seen from the perspective of FIG. 23, the sealing part 2110 is trapezoidal. The first frame 2111 includes a distal disc surface facing the anchoring part 2120, a proximal disc surface facing away from the anchoring part 2120, and a waist 2113 connected between the distal disc surface and the proximal disc surface. The proximal disc surface and the distal disc surface are generally planar. The waist is connected between the proximal disc surface and the distal disc surface and in the shape of a cone.

In this embodiment, the first conductive portion 2150 is at least part of the first frame 2111, and the first conductive portion 2150 is at least provided on the edge area of the sealing part 2110 with a large radial dimension, such as on the waist 2113. Preferably, the first conductive portion 2150 is provided on the portion of the sealing part 2110 with the maximum radial dimension in the circumferential direction. As shown in FIG. 23, the first conductive portion 2150 can be provided on the waist 2113 and the proximal disc surface. In some embodiments, the first conductive portion 2150 can be provided on at least part of the waist 2113, the proximal disc surface and the distal disc surface. The surface of the first frame 2111 that is not used as the first conductive portion 2150 is subjected to insulation treatment.

A connecting piece 2130 at the proximal end of the sealing part 2110 can be used for mechanical connection and electrical connection with the delivery device.

The second conductive portion 2160 is disposed on the anchoring part 2120, and specifically is a wave-like electrode. The second frame 2121 can be insulated from the second conductive portion 2160 or not.

A connecting piece 2190 is connected between the sealing part 2110 and the anchoring part 2120. In some embodiments, the connecting piece 2190 is configured for an insulation connection between the first conductive portion 2110 and the second conductive portion 2120. In some embodiments, the connecting piece 2190 is configured to be electrically connected between the second conductive portion 2160 and the delivery device.

The anchoring part 2120 mainly differs from the anchoring part 2020 in that the ends of two adjacent fourth anchoring struts 2125 away from the third anchoring struts 2124 are connected together to improve the mechanical properties of the anchoring part 2120, preventing the fourth anchoring struts 2125 from interlocking with each other during the radial deformation of the left atrial appendage occlusion and ablation device 2100, and improving the reliability of the left atrial appendage occlusion and ablation device 2100.

### Twenty-second embodiment

Referring to FIG. 24, the ablation system according to this embodiment includes a left atrial appendage occlusion and ablation device 2200, which is configured to occlude and ablate the left atrial appendage. The left atrial appendage occlusion and ablation device 2200 includes a sealing part 2210 disposed at the proximal side and an anchoring part 2220 disposed at the distal side, and the sealing part 2210 and the anchoring part 2220 are connected to each other.

The main difference between the left atrial appendage occlusion and ablation device 2200 according to this embodiment and the left atrial appendage occlusion and ablation device 2100 according to the twenty-first embodiment is that the first conductive portion 2250 is an electrode element disposed on the portion of the sealing part 2110 with the maximum radial dimension in the form of a ring electrode. The second conductive portion 2260 is an electrode element disposed on the surface of the anchoring part 2220, and the electrode element specifically includes a plurality of dot-shaped electrodes arranged at intervals. In this embodiment, two dot-shaped electrodes are arranged at an interval on each anchoring strut 2224. In some other embodiments, the number of dot-shaped electrodes arranged on each anchoring strut 2224 can be adjusted as required, and some third anchoring struts 2224 may not be provided with dot-shaped electrodes.

### Twenty-third embodiment

Referring to FIG. 25, the ablation system according to this embodiment includes a left atrial appendage occlusion and ablation device 2300, which is configured to occlude and ablate the left atrial appendage. The left atrial appendage occlusion and ablation device 2300 includes a sealing part 2310 disposed at the proximal side and an anchoring part 2320 disposed at the distal side, and the sealing part 2310 and the anchoring part 2320 are connected to each other.

The main difference between the left atrial appendage occlusion and ablation device 2300 according to this embodiment and the left atrial appendage occlusion and ablation device 2100 according to the twenty-first embodiment is that the specific structures of the sealing part 2310 and the anchoring part 2320 are different from that in the twenty-first embodiment.

Specifically, in this embodiment, both the sealing part 2310 and the anchoring part 2320 are made by weaving.

The first frame 2311 of the sealing part 2310 has a double-layer disc structure, including a distal disc facing the anchoring part 2320 and a proximal disc facing away from the anchoring part 2320. The proximal disc surface is generally planar, the distal disc surface is partially spherical, and the proximal disc surface and the distal disc surface are smoothly transitioned and connected to each other. The first conductive portion 2350 is at least part of the first frame 2311 and provided in the edge area of the sealing part 2310 with a large radial dimension.

The second frame 2321 of the anchoring part 2320 has a double-layer disc structure, which is generally cylindrical, and the radial dimension of the distal side wall of the second frame 2321 is smaller than the radial dimension of the proximal side wall of the second frame 2321. The second conductive portion 2360 is an electrode element disposed on the anchoring part 2320 in the form of a wave-like electrode.

### Twenty-fourth embodiment

Referring to FIG. 26, the ablation system according to this embodiment includes a left atrial appendage occlusion and ablation device 2400, which is configured to occlude and ablate the left atrial appendage. The left atrial appendage occlusion and ablation device 2400 includes a sealing part 2410 disposed at the proximal side and an anchoring part 2420 disposed at the distal side, and the sealing part 2410 and the anchoring part 2420 are connected to each other.

The main difference between the left atrial appendage occlusion and ablation device 2400 according to this embodiment and the left atrial appendage occlusion and ablation device 2300 according to the twenty-third embodiment is that the first conductive portion 2450 is an electrode element disposed on the surface of the first frame 2411 of the sealing part 2410 in the form of a ring electrode. For the detail of the ring electrode, please refer to the above-mentioned embodiment.

### Twenty-fifth embodiment

Referring to FIG. 27, the ablation system according to this embodiment includes a left atrial appendage occlusion and ablation device 2500, which is configured to occlude and ablate the left atrial appendage. The left atrial appendage occlusion and ablation device 2500 includes a sealing part 2510 disposed at the proximal side and an anchoring part 2520 disposed at the distal side, and the sealing part 2510 and the anchoring part 2520 are connected to each other.

The main difference between the left atrial appendage occlusion and ablation device 2500 according to this embodiment and the left atrial appendage occlusion and ablation device 2300 according to the twenty-third embodiment is that the structure of the anchoring part 2520 and the structure of the second conductive portion 2560 are different from that in the twenty-third embodiment.

Specifically, the anchoring part 2520 is made by cutting. The anchoring part 2520 is a double-layer disc structure, with closed proximal and distal ends. The second frame 2521 includes first anchoring struts 2522, second anchoring struts 2523 and third anchoring struts 2524 connected sequentially from the distal end to the proximal end .

The first anchoring strut 2522, the second anchoring strut 2523 and the third anchoring strut 2524 all extend between the proximal end and the distal end. The proximal end of each first anchoring strut 2522 is connected to two different second anchoring struts 2523 which extend in different directions. Within four second anchoring struts 2523 connected to two adjacent first anchoring struts 2522, the middle sections of two adjacent second anchoring struts 2523 connected to different first anchoring struts 2522 are closely connected to each other. The two second anchoring struts 2523 connected to the same first anchoring strut 2522 are connected to each other at the distal ends, and are connected to the distal end of the third anchoring strut 2524. The proximal ends of the plurality of third anchoring struts 2524 are joined together, so as to form the anchoring part 120 with two closed ends.

The plurality of second anchoring struts 2523 define a plurality of meshes for the anchoring part 2520, so as to improve the radial deformation ability of the anchoring part 2520 and increase the friction between the anchoring part 2520 and the tissue.

The second conductive portion 2560 is an electrode element disposed on the surface of the second frame 2521 of the anchoring part 2520. Specifically, the electrode element is in the form of a ring electrode. For the detail of the ring electrode, please refer to the above-mentioned embodiment.

### Twenty-sixth embodiment

Referring to FIG. 28, the ablation system according to this embodiment includes a left atrial appendage occlusion and ablation device 2600, which is configured to occlude and ablate the left atrial appendage. The left atrial appendage occlusion and ablation device 2600 includes a sealing part 2610 disposed at the proximal side and an anchoring part 2620 disposed at the distal side, and the sealing part 2610 and the anchoring part 2620 are connected to each other.

The main difference between the left atrial appendage occlusion and ablation device 2600 according to this embodiment and the left atrial appendage occlusion and ablation device 2500 according to the twenty-fifth embodiment is that the structure of the second conductive portion 2660 is different from that in the twenty-fifth embodiment.

The second conductive portion 2660 is an electrode element disposed on the surface of the second frame 2621 of the anchoring part 2620. Specifically, the electrode element includes a plurality of dot-shaped electrodes disposed on the surface of the second frame 2621 at intervals. For the detail of the dot-shaped electrode, please refer to the above-mentioned embodiment.

### Twenty-seventh embodiment

Referring to FIG. 29, the ablation system according to this embodiment includes a left atrial appendage occlusion and ablation device 2700, which is configured to occlude and ablate the left atrial appendage. The left atrial appendage occlusion and ablation device 2700 includes a sealing part 2710 disposed at the proximal side and an anchoring part 2720 disposed at the distal side, and the sealing part 2710 and the anchoring part 2720 are connected to each other.

The main difference between the left atrial appendage occlusion and ablation device 2700 according to this embodiment and the left atrial appendage occlusion and ablation device 1500 according to the fifteenth embodiment is that the structure of the sealing part 2710 and the structure of the first conductive portion 2750 are different from that in the fifteenth embodiment.

Specifically, the first frame 2711 of the sealing part 2710 includes a plurality of first sealing struts, and two ends of each first sealing strut are both arranged at the central axis of the sealing part 2710. In a projection plane perpendicular to the central axis, or in view of the direction from the distal end to the proximal end, or from the proximal end to the distal end, each first sealing strut forms a generally ring-shaped support ring. In this embodiment, the ring is sector-shaped. In other embodiments, the ring can be a circular ring, an elliptical ring, a partial ring, or other irregular rings. A plurality of supporting rings formed by a plurality of first sealing struts are arranged in the circumferential direction to form the first frame 2711.

As shown in FIG. 29, adjacent supporting rings overlap each other in the circumferential direction. In a projection plane perpendicular to the central axis, or in view of the direction from the distal end to the proximal end, or from the proximal end to the distal end, the central angles corresponding to adjacent support rings overlap each other. In other embodiments, the central angles corresponding to adjacent supporting rings do not overlap. In some embodiments, adjacent supporting rings are spaced from each other by a central angle.

As shown in FIG. 29, the two ends of each first sealing strut have different axial levels, that is, the two ends of each first sealing strut are axially offset from each other. In other embodiments, the two ends of each first sealing strut have the same axial level.

The first conductive portion 2750 is part of the first frame 2711 of the sealing part 2710. Specifically, the first conductive portion 2750 is the portion of the first frame 2711 with a large radial dimension, including the portion of the first frame 2711 with the maximum radial dimension. In some embodiments, the first conductive portion 2750 is provided at the portion of the first frame 2711 with the maximum radial dimension, i.e., a circle of frame at the peripheral edge of the first frame 2711.

### Twenty-eighth embodiment

Referring to FIG. 30, the ablation system according to this embodiment includes a left atrial appendage occlusion and ablation device 2800, which is configured to occlude and ablate the left atrial appendage. The left atrial appendage occlusion and ablation device 2800 includes a sealing part 2810 disposed at the proximal side and an anchoring part 2820 disposed at the distal side, and the sealing part 2810 and the anchoring part 2820 are connected to each other.

The main difference between the left atrial appendage occlusion and ablation device 2800 according to this embodiment and the left atrial appendage occlusion and ablation device 2700 according to the twenty-seventh embodiment is that the structure of the first conductive portion 2850 is different from that in the twenty-seventh embodiment.

In this embodiment, the first conductive portion 2850 is an electrode element disposed at the portion of the sealing part 2810 with the maximum radial dimension in the circumferential direction in the form of a ring electrode. For the detail of the ring electrode, please refer to the above-mentioned embodiment.

### Twenty-ninth embodiment

Referring to FIG. 31, the ablation system according to this embodiment includes a left atrial appendage occlusion and ablation device 2900, which is configured to occlude and ablate the left atrial appendage. The left atrial appendage occlusion and ablation device 2900 includes a sealing part 2910 disposed at the proximal side and an anchoring part 2920 disposed at the distal side, and the sealing part 2910 and the anchoring part 2920 are connected to each other.

The main difference between the left atrial appendage occlusion and ablation device 2900 according to this embodiment and the left atrial appendage occlusion and ablation device according to the previous embodiments is that the anchoring part 2920 has a double-disc structure.

Specifically, the sealing part 2910 and the anchoring part 2920 are both made by weaving wire.

The sealing part 2910 is cylindrical or conical, and its radial dimension is greater than that of the ostium of the left atrial appendage. As shown in the figure, the first conductive portion 2950 is a ring electrode disposed on the portion of the sealing part 2910 with the maximum radial dimension. In some embodiments, the first conductive portion 2950 may be disposed on other portions in the edge area.

The anchoring part 2920 includes a first anchoring part 2922 and a second anchoring part 2923 spaced apart from each other in the axial direction. The first anchoring part 2922 and the second anchoring part 2923 are both made by weaving wire and are cylindrical or conical. A connecting piece 2924 is disposed between the first anchoring part 2922 and the second anchoring part 2923. In some embodiments, the connecting piece 2924 is flexible and/or elastic.

The second conductive portion 2960 is an electrode element disposed at the portion of the first anchoring part 2922 with the maximum radial dimension. Specifically, the electrode element is a ring electrode. For the detail of the ring electrode, please refer to the above-mentioned embodiment.

In some embodiments, the second conductive portion 2960 may be disposed on the second anchoring part 2923, or the portion of the sealing part 2910 with a large radial dimension, or the connecting piece 2924.

### Thirtieth Embodiment

Referring to FIG. 32, the ablation system according to this embodiment includes a left atrial appendage occlusion and ablation device 3000, which is configured to occlude and ablate the left atrial appendage. The left atrial appendage occlusion and ablation device 3000 includes a sealing part 3010 disposed at the proximal side and an anchoring part 3020 disposed at the distal side, and the sealing part 3010 and the anchoring part 3020 are connected to each other.

The main difference between the left atrial appendage occlusion and ablation device 3000 according to this embodiment and the left atrial appendage occlusion and ablation device according to the twenty-ninth embodiment is the structures of the first conductive portion 3050 and the second conductive portion 3060.

In this embodiment, the first conductive portion 3050 is at least part of the first frame of the sealing part 3010, preferably the portion of the sealing part 3010 with a large radial dimension, more preferably the portion of the sealing part 3010 with the maximum radial dimension.

The second conductive portion 3060 is at least part of the frame of the anchoring part 3020, preferably the portion of the first anchoring part 3022 with a large radial dimension, more preferably the portion of the first anchoring part 3022 with the maximum radial dimension. The second conductive portion 3060 may alternatively be provided on the second anchoring part 3023, or on the sealing part 3010, or on the connecting piece 3024.

### Thirty-first embodiment

Referring to FIG. 33, the ablation system according to this embodiment includes a left atrial appendage occlusion and ablation device 3100, which is configured to occlude and ablate the left atrial appendage. The left atrial appendage occlusion and ablation device 3100 includes a sealing part 3110 disposed at the proximal side and an anchoring part 3120 disposed at the distal side, and the sealing part 3110 and the anchoring part 3120 are connected to each other.

The main difference between the left atrial appendage occlusion and ablation device 3100 according to this embodiment and the left atrial appendage occlusion and ablation device according to the thirtieth embodiment is the structures of the sealing part 3110 and the anchoring part 3120, and the structures of the first conductive portion 3150 and the second conductive portion 3160.

Both the sealing part 3110 and the anchoring part 3120 can be made by weaving. The sealing part 3110 is in the shape of a double-layer meshed disc, and the diameter of the sealing part 3110 is greater than that of the ostium of the left atrial appendage.

The anchoring part 3120 includes the first anchoring part 3122 and a second anchoring part 3123. The first anchoring part 3122 and the second anchoring part 3123 can be in the shape of a single-layer meshed disc or a multi-layer meshed disc. The first anchoring part 3122 is disposed at the proximal side of the second anchoring part 3123.

A connecting piece 3124 and a connecting piece 3125 are connected between the sealing part 3110 and the first anchoring part 3122. The connecting piece 3124 and the connecting piece 3125 extend between the proximal end and the distal end. The connecting piece 3124 is arranged along the axial direction of the left atrial appendage occlusion and ablation device 3100. At least one connecting piece 3125 is provided around the connecting piece 3124 in the circumferential direction.

The connecting piece 3124 can be a helical structure so as to have a variable length, which enables the left atrial appendage occlusion and ablation device 3100 to be applied to left atrial appendages of different shapes and sizes. The connecting piece 3124 is bendable, so that the left atrial appendage occlusion and ablation device 3100 can assume different angles and different orientations, which also enables the left atrial appendage occlusion and ablation device 3100 to be applied to left atrial appendages of different shapes and sizes. The connecting piece 3124 has a substantially constant or constant cross-section.

The connecting piece 3125 is made of a substantially inelastic or completely inelastic material such that the connecting piece 3125 can prevent the connecting piece 3124 from stretching beyond a predetermined length. Since the connecting piece 3125 can prevent the connecting piece 3124 from stretching beyond the predetermined length, the connecting piece 3125 can prevent the connecting piece 3124 from being overstretched during loading the left atrial appendage occlusion and ablation device 3100 into the left atrial appendage or removing the left atrial appendage occlusion and ablation device 3100 from the left atrial appendage. Two connecting pieces 3125 or more connecting pieces 3125 may be arranged around the connecting piece 3124.

The first conductive portion 3150 is at least part of the first frame 3111 of the sealing part 3110, preferably a portion of the sealing part 3110 with a large radial dimension, more preferably a portion of the sealing part 3110 with the maximum radial dimension.

The second conductive portion 3160 is at least part of the second frame of the second anchoring part 3123, preferably a portion of the second anchoring part 3123 with a large radial dimension, more preferably a portion of the second anchoring part 3123 with the maximum radial dimension

In some embodiments, the second conductive portion 3160 is at least part of the second frame of the first anchoring part 3122, or is provided on the connecting piece 3124 and/or the connecting piece 3125.

The connecting piece 3124 and the connecting piece 3125 can be configured as conductive connecting pieces for transmitting electric energy.

### Thirty-second embodiment

Referring to FIG. 34, the ablation system according to this embodiment includes a left atrial appendage occlusion and ablation device 3200, which is configured to occlude and ablate the left atrial appendage. The left atrial appendage occlusion and ablation device 3200 includes a sealing part 3210 disposed at the proximal side and an anchoring part 3220 disposed at the distal side, and the sealing part 3210 and the anchoring part 3220 are connected to each other.

The main difference between the left atrial appendage occlusion and ablation device 3200 according to this embodiment and the left atrial appendage occlusion and ablation device 3100 according to the thirty-first embodiment is the structure of the second conductive portion 3260.

The second conductive portion 3260 is an electrode element disposed on the surface of the first anchoring part 3222 in the form of a ring electrode. Preferably, the second conductive portion 3260 is disposed on a portion of the first anchoring part 3222 with a large radial dimension, more preferably, on a portion of the first anchoring part 3222 with the maximum radial dimension. In some embodiments, the second conductive portion 3260 is an electrode element disposed on the surface of the second anchoring part 3223, preferably, on the portion of the second anchoring part 3223 with a large radial dimension, more preferably, on the portion of the second anchoring part 3223 with the maximum radial dimension.

### Thirty-third embodiment

FIG. 35 is a schematic view of an ablation system 3300 according to the thirty-third embodiment, and FIG. 36 is a schematic view of the ablation system 3300 according to the thirty-third embodiment implanted in the left atrial appendage. Referring to FIG. 35 and FIG. 36, the ablation system 3300 includes a support 3310, a delivery device 3320 and an ablation element 3330. The support 3310 is configured to be implanted into the target tissue to be ablated, such as the ostium of the left atrial appendage, the ostium of the pulmonary vein, etc.. The delivery device 3320 is configured to deliver the support 3310 to the target tissue, such as the ostium of the left atrial appendage or other target tissues which include but are not limited to myocardial tissue. The ablation element 3330 is configured to electrically ablate the wall of the target tissue.

The support 3310 has a single-disk structure and is generally columnar. The support 3310 is a hollow mesh made by weaving and/or cutting. The support 3310 includes a sealing part 3311 and an anchoring part 3312 connected with each other. After the support 3310 is released to the left atrial appendage 10, the sealing part 3311 is located at the distal side of the ostium of the left atrial appendage. The distal side of the anchoring part 3312 defines an opening.

Anchoring barbs 3313 are provided on the anchoring part 3312 to be anchored into the inner wall of the left atrial appendage. A plurality of anchoring barbs 3313 are evenly arranged along the outer wall of the anchoring part 3312. The anchoring barbs 3313 can be provided differently depending on the structures thereof. The anchoring barb 3313 can be directly fixed on the anchoring part 3312, specifically, by welding. Alternatively, the anchoring barb 3313 can be fixedly connected to the anchoring part 3312 through a steel sleeve. Alternatively, the anchoring barb 3313 and the anchoring part 3312 can be formed in one piece, that is, the anchoring barb 3313 can be formed by directly extending from the anchoring part 3312.

The ablation element 3330 includes a first conductive portion 3331 and a second conductive portion 3332. The first conductive portion 3331 and the second conductive portion 3332 are configured to transmit pulse ablation energy with different polarities to the target tissue, so as to realize tissue ablation. In this embodiment, the first conductive portion 3331 is provided on the support 3310, and is electrically connected to an external pulse ablation source through the delivery device 3320. The second conductive portion 3332 is provided on the delivery device 3320 and movable relative to the support 3310, and is configured to be released on the proximal side of the support 3310. Specifically, the side wall of the support 3310 is arc-shaped, and the portion of the support 3310 with the maximum radial dimension is configured to radially abut against the tissue on the inner wall of the left atrial appendage and can contact the wall closely. The first conductive portion 3331 is provided on the portion of the support 3310 with the maximum radial dimension, which facilitates the first conductive portion 3331 to contact the wall closely to improve the ablation effect. The portion of the support 3310 with the maximum radial dimension is located between the proximal end and the distal end thereof, and close to the proximal end. After the support 3310 is implanted into the left atrial appendage 10, the first conductive portion 3331 is located inside the left atrial appendage 10. Since the support 3310 has a single-disc structure, and the portion of the support 3310 with the maximum radial dimension is located between the proximal end and the distal end of the support 3310, it is convenient for the support 3310 to be implanted and fixed in the inner cavity of the left atrial appendage. In a modified embodiment, the side wall of the support has the same radial dimension at different axial levels.

In some embodiments, at least part of the first conductive portion 3331 and/or the second conductive portion 3332 can be configured to collect physiological signals of the tissue, that is, the ablation element 3330 can be used for both ablation and mapping. In some embodiments, part of the first conductive portion 3331 may only be used for mapping, and cannot be used for ablation. In some embodiments, part of the first conductive portion 3331 may be used for mapping, and the other may be used for ablation. In some embodiments, at least part of the first conductive portion 3331 may be used for mapping and ablation, respectively, in different periods. In some embodiments, part of the second conductive portion 3332 may only be used for mapping, and cannot be used for ablation. In some embodiments, part of the second conductive portion 3332 may be used for mapping, and the other may be used for ablation. In some embodiments, at least part of the second conductive portion 3332 may be used for mapping and ablation, respectively, in different periods.

The delivery device 3320 includes an inner sheath 3321 and an ablation catheter 3323. The inner sheath 3321 is connected with the support 3310. The delivery device 3320 further includes an outer sheath 3322 surrounding the inner sheath 3321. The outer sheath 3322 and the inner sheath 3321 can move relative to each other. The distal end of the inner sheath 3321 is connected to the support 3310. The proximal end of the ablation catheter 3323 is connected to the distal end of the outer sheath 3322. After ablation, the ablation catheter 3323 can be recovered in an outer catheter (not shown) surrounding the outer sheath 3322. The ablation catheter 3323 is configured to be released on the proximal side of the sealing part 3311. The second conductive portion 3332 is provided on the ablation catheter 3323. Specifically, the ablation catheter 3323 includes a plurality of support struts 3324 disposed on the distal side of the outer sheath, and the plurality of support struts 3324 are umbrella-shaped after being released on the proximal side of the support 3310, forming an umbrella-shaped frame.

Specifically, the ablation catheter 3323 includes a plurality of support struts 3324 arranged in a radial pattern around the axis of the ablation catheter 3323. The proximal ends of the plurality of support struts 3324 are joined together with the distal end of the outer sheath 3322. After the support struts 3324 are released, each support strut 3324 is in the shape of an arc protruding toward the support 3310. The end of each support strut 3324 away from its proximal end is a free end, and the free end is configured to extend proximally. The second conductive portion 3332 is provided on the support struts 3324.

The second conductive portion 3332 may be parts of the support struts 3324 or the entire support struts 3324, or an electrode element(s) disposed on the support struts 3324. In this embodiment, the end of each support strut 3324 is provided with an electrode element. The radial dimension of the second conductive portion 3332 is relatively large and larger than the radial dimension of the first conductive portion 3331, so that the shortest electric field line E can pass through the tissue at the ostium. It can be understood that the number of the electrode elements on the support struts 3324 can be adjusted as required. Alternatively, some support struts 3324 may not be provided with electrode element.

Since the second conductive portion 3332 is provided on the peripheral edge area of the ablation catheter with a large radial dimension, it is convenient for the second conductive portion 3332 to contact the tissue proximal to the ostium of the left atrial appendage after being released. The portion of the support 3310 with the maximum radial dimension is located between the proximal end and the distal end with the maximum radial dimension, so that when the support 3310 is implanted into the left atrial appendage, the sealing part of the support 3310 is implanted into the distal side of the ostium of the left atrial appendage, without covering the ostium of the left atrial appendage, so as to avoid mutual interference between the support 3310 and the umbrella-shaped frame. In the natural state (no external force), or in the ablation process, the radial dimension of the ring structure formed by the second conductive portion 3332 is greater than the radial dimension of the ring structure formed by the first conductive portion 3331, that is, within the first conductive portion and the second conductive portion, the radial dimension of the ring structure of the proximal conductive portion is greater than the radial dimension of the ring structure of the distal conductive portion, so that the shortest electric field line E is inclined, facilitating at least one shortest electric field line between the first conductive portion and the second conductive portion to pass through the tissue of the left atrial appendage outside the accommodating part, thereby ensuring the ablation effect. In this embodiment, the accommodating part includes the portions of the support 3310 and the support struts 3324 facing and close each other.

It would be appreciated that the first conductive portion 3331 and the second conductive portion 3332 can be various forms of electrodes disposed on the support 3310 or the support struts 3324, for example, at least one of dot-shaped electrodes, rod-shaped electrodes, arc electrodes, ring electrodes, and wave-like electrodes. Alternatively, the first conductive portion 3331 can be at least part of the support 3310, and the second conductive portion 3332 can be at least part of the support struts 3324. For example, the support 3310 may have a hollow mesh made of conductive metal material by weaving and/or cutting, and the first conductive portion 3331 is at least part of the support 3310 or the entire support 3310. In some embodiments, the support struts 3324 of the ablation catheter 3323 may be made of conductive metal material by weaving and/or cutting, and the second conductive portion 3332 is at least part of the support struts 3324 or the entire support struts 3324.

Both the first conductive portion 3331 and the second conductive portion 3332 can include a plurality of electrodes. As shown in FIG. 35, the support 3310 is provided with a plurality of first conductive portions 3331, specifically, two first conductive portions 3331, which are respectively provided on the sealing part 3311 and the anchoring part 3312. It can be understood that the plurality of first conductive portions 3331 may be provided on the sealing part 3311 or the anchoring part 3312. The plurality of electrodes of the first conductive portion 3331 may be arranged at intervals or be in contact with each other. The plurality of electrodes of the second conductive portion 3332 may be arranged at intervals or connected to each other.

Compared with the prior art, the ablation system 3300 disclosed here can achieve pulse ablation on the target tissue through the cooperation between the first conductive portion 3331 and the second conductive portion 3332.

In the following, the ablation system 3300 is used as an example of left atrial appendage occlusion and ablation system for illustration, that is, the ablation system 3300 is configured for occlusion and ablation of the left atrial appendage 10.

The left atrial appendage occlusion and ablation system includes a left atrial appendage occlusion and ablation device and a delivery device 3320. The left atrial appendage occlusion and ablation device includes a support 3310 and a first conductive portion 3331 of the ablation element 3330. The support 3310 is connected to the delivery device 3320, and the first conductive portion 3331 is connected to the pulse ablation instrument through the delivery device 3320. In some embodiments, the first conductive portion 3331 is connected to the pulse ablation instrument through the delivery device 3320 via a cable. In some embodiments, a flow blocking film is provided on the support 3310 to prevent the thrombus inside the left atrial appendage from flowing out to the left atrium.

In some embodiments, both the first conductive portion 3331 and the second conductive portion 3332 are provided on the support 3310, and the two conductive portions are configured to transmit ablation electric energy with the same or different parameters, for example, to transmit ablation electric energy with different polarities. In this embodiment, the support 3310 is provided with the first conductive portion, and the delivery device 3320 is provided with the second conductive portion, for example, on the ablation catheter of the delivery device 3320 provided at the proximal side of the support 3310. In other embodiments, the ablation catheter provided with the second conductive portion is configured to be released on the distal side of the support 3310. In some embodiments, the second conductive portion is provided independently from the support 3310 and the delivery device 3320, and is configured to contact the surface of the living body during the ablation process.

In this embodiment, the first conductive portion and the second conductive portion meet the parameters set in the previous embodiments. For example, during the ablation, the intensity of the electric field generated by the first conductive portion and the second conductive portion at a distance of 3 mm from the respective surfaces thereof is greater than 400 V/cm. In some embodiments, the ablation system 3300 is configured to ablate cardiac tissue other than the left atrial appendage 10, for example, the pulmonary vein which generally has a thickness of 2 mm. During the ablation process, the intensity of the electric field generated by the ablation element 3330 at a distance of 2 mm from its surface is greater than the field intensity threshold of the myocardium, for example, is greater than 400 V/cm, thereby achieving transmural ablation.

In the case where the ablation element 3330 is provided on the support 3310 and/or the delivery device, the pulse voltage for the ablation element 3330 (the first conductive portion and the second conductive portion) is in the range of 500 V to 4000 V, the discharge area of the first conductive portion 3331 is in the range of 15 mm² to 4700 mm², and the discharge area of the second conductive portion 3332 is in the range of 15 mm² to 4700 mm². In the case where the conductive portion includes a plurality of electrode elements, the discharge area refers to the sum of the discharge areas of all the electrode elements or the frame of the conductive portion. The distance between two closest points on the surfaces of the first conductive portion 3331 and the second conductive portion 3332 ranges from 1 mm to 45 mm. Preferably, the distance between the first conductive portion 3331 and the second conductive portion 3332 can be further limited to the range of 3 mm to 22 mm. Within this range, the ablation zones of the two conductive portions can partially overlap with each other. The ablation system is configured to ablate myocardial tissue. The zone where the intensity of the electric field generated around the first conductive portion 3331 is greater than the intensity threshold of the myocardium is the first ablation zone, and the zone where the intensity of the electric field generated around the second conductive portion 3332 is greater than the intensity threshold of the myocardium is the second ablation zone. The ablation zone formed after the two ablation zones partially overlap in the axial direction is relatively large.

The support 3310 in FIG. 35 is provided with two first conductive portions 3331. In some embodiments, one of two first conductive portions 3331 can be used as required.

When the frequency ranges from 500 Hz to 3 MHz and the voltage ranges from 500 V to 4000 V, and the ablation zones surrounding the surfaces of the respective conductive portions have electric field intensity greater than or equal to 400 V/cm, the axial dimension of the ablation zone formed by the two ablation zones partially overlapping with each other in the axial direction is greater than that of any single ablation zone, so that the energy of the two ablation zones can be concentrated to ablate one site, improving the ablation success rate of the area to be ablated. That is to say, after the device is implanted into the ostium of the left atrial appendage 11, even the peripheral edge area of the sealing part 3311 contacts the ostium loosely at some positions, since the ablation element 3330 has a relatively large ablation zone in the axial direction, it is easier to achieve transmural ablation around the sealing part 3311 than two spaced ablation zones with smaller axial dimensions.

The ablation zones partially overlapping with each other include proximal and distal parts in the axial direction. Even if the proximal part cannot contact the wall closely and its peripheral edge is difficult to cover the outer wall of the left atrial appendage, the distal part can cover the outer wall of the left atrial appendage, thereby reducing the influence of the released sealing part 3311 contacting the wall loosely on the ablation effect of the occlusion and ablation system 3300, and improving the success rate of ablation of the system.

In this embodiment, the first conductive portion 3331 is provided on the sealing part 3311, and the ablation zone formed by the first ablation zone and the second ablation zone partially overlapping with each other is distributed at the ostium of the left atrial appendage 11, so as to ablate the ostium of the left atrial appendage 11. Compared with the inner tissue of the left atrial appendage, the tissue at the ostium of the left atrial appendage has a smooth surface and a regular shape, so that the first conductive portion 3331 is more likely to contact the wall, and the tissue at the ostium of the left atrial appendage has a relatively even thickness, so that the ablation zones can achieve continuous transmural ablation at the ostium.

Since the wall thickness of the left atrial appendage is generally 3 mm, and the distance between the boundary of the two overlapping ablation zones and the surfaces of the conductive portions is larger than the wall thickness of the left atrial appendage, so transmural ablation can be achieved.

Referring to FIG. 35 and FIG. 36, the operation method of the ablation system 3300 in this embodiment includes the following steps:

Step A: delivering the left atrial appendage occlusion and ablation device with the delivery device 3320 to the ostium of the left atrial appendage and releasing the same to occlude the ostium of the left atrial appendage. Specifically, after the left atrial appendage occlusion and ablation device is released to the left atrial appendage 10, the inner sheath 3321 remains connected to the occlusion and ablation device, and the second conductive portion 3332 (ablation catheter 3323) is released; when the second conductive portion 3332 contacts the tissue proximal to the ostium of the left atrial appendage, the position of the ablation catheter 3323 is fixed, and the pulse ablation source is powered on at the same time; under the action of the pulse energy, at least one shortest electric field line E formed by the first conductive portion 3331 and the second conductive portion 3332 passes through the tissue at the ostium, facilitating the transmural ablation at the ostium, thereby destroying the intracellular and extracellular homeostasis and achieving the effect of electrical isolation, ensuring that no electrophysiological signals can be transmitted between the left atrial appendage 10 and the left atrium 12 located on two sides of the target tissue, so as to treat atrial fibrillation. After the ablation is completed, the connection cable is released.

Step B: withdrawing the ablation catheter 3323 to complete the operation.

Step C: performing ablation through the ablation catheter 3323 alone. Specifically, under the guidance of DSA contrast or ultrasound, the ablation catheter 3323 is positioned at the ostium of the left atrial appendage 11, and the second conductive portion 3332 is positioned to contact the tissue proximal to the ostium of the left atrial appendage. The position of the ablation catheter 3323 is fixed, and the pulse ablation source is powered on at the same time. The electrodes of the second conductive portion 3332 are configured to transmit pulse ablation energy with different polarities. The plurality of electrodes of the second conductive portion 3332 assume a ring shape, so as to form a transmural ring-like ablation zone in the left atrial appendage 10, at the ostium of the left atrial appendage 11 and the proximal side of the ostium of the left atrial appendage, the pulmonary vein, and the atrium.

Step C can be performed before step A, and/or between step A and step B. The release sequence of the ablation catheter 3323 and the occlusion and ablation device can be adjusted as required, and the release sequence of the two has no mutual restriction. In some embodiments, step C can be omitted.

### Thirty-fourth embodiment

FIG. 37 is a schematic view of an ablation system 3400 according to a thirty-fourth embodiment of the present disclosure.

The ablation system 3400 in this embodiment is similar to the ablation system 3300 in the thirty-third embodiment. The similarities therebetween refer to the above description in the thirty-third embodiment, and would not be repeated here. The main difference between this embodiment and the thirty-third embodiment is the structure of the ablation catheter.

In this embodiment, the ablation catheter 3423 includes an outer sheath 3422 and a plurality of support struts disposed at the distal end of the outer sheath 3422. The first conductive portion 3431 is provided on the proximal outer peripheral wall of the support 3410. The second conductive portion 3432 is provided on the plurality of support struts, and the second conductive portion 3432 can be parts of the plurality of support struts, or the entire of the plurality of support struts, or electrode elements arranged on the plurality of support struts.

Each of the plurality of support struts forms a support ring, and the plurality of support rings are sequentially distributed in the circumferential direction. Each support ring has a ring-shaped projection on a plane perpendicular to the axis of the support. In this embodiment, the ring is sector-shaped. In other embodiments, the ring can be a circular ring, an elliptical ring, a partial ring, or other regular or irregular rings. The projections of adjacent supporting rings on the plane perpendicular to the axis of the support are partially overlapped. In other embodiments, the projections of adjacent support rings on the plane do not overlap each other, or the overlapping area is smaller or larger.

The two ends of the support strut have different axial levels, that is, the two ends of the support strut are axially offset from each other, thereby increasing the supporting performance of each support ring. In some embodiments, the two ends of the support ring have the same axial level, and may be connected together or spaced apart.

The edge area of the support ring can contact the tissue wall closely to ensure the effect of ablation and mapping.

In some embodiments, the support 3410 has a single-disc or multiple-disc structure, and the second conductive portion 3432 is disposed on the delivery device 3420. The second conductive portion 3432 is movable relative to the support 3410, and is configured to be released on the proximal side of the support 3410. The first conductive portion is disposed adjacent to the proximal end of the support 3410. The distance between the first conductive portion and the second conductive portion 3432 is adjusted to be short, so that the ablation catheter on the distal side of the delivery device 3420 can cooperate with the first conductive portion on the sealing part well.

### Thirty-fifth embodiment

FIG. 38 is a schematic view of an ablation system 3500 according to the thirty-fifth embodiment of the present disclosure.

The ablation system 3500 in this embodiment is similar to the ablation system 3400 in the thirty-fourth embodiment. The similarities therebetween refer to the above description in the thirty-fourth embodiment, and would not be repeated here. The main difference between this embodiment and the thirty-fourth embodiment is the structure of the ablation catheter.

In this embodiment, the ablation catheter 3523 includes a meshed disc structure disposed at the distal end of the outer sheath 3522, and the meshed disc structure can be made by weaving or cutting. As shown in FIG. 38, the meshed disc structure is a double-layer meshed disc, that is, the meshed disc structure includes two layers of woven meshes in the axial direction (distributed in the up-and-down direction as shown in the figure) of the meshed disc structure, and the two layers of woven meshes are connected to each other at the peripheral edge. In some other embodiments, the meshed disc is a single-layer meshed disc, that is, the meshed disc only includes a single-layer woven mesh in the axial direction (in the up-and-down direction as shown in the figure) of the meshed disc structure.

The second conductive portion 3532 is provided on the woven meshed disc. The first conductive portion 3531 is provided on the proximal outer peripheral wall of the support 3510. The second conductive portion 3532 can be part of the meshed disc structure, or the entire meshed disc structure so that the whole woven meshed disc can be used as an electrode to transmit ablation energy to the tissue. In some embodiments, the second conductive portion 3532 may be an electrode disposed on the meshed disc structure.

### Thirty-sixth embodiment

FIG. 39 is a schematic view of an ablation system 3600 according to the thirty-sixth embodiment of the present disclosure.

The ablation system 3600 in this embodiment is similar to the ablation system 3300 in the thirty-third embodiment. The similarities therebetween refer to the above description in the thirty-third embodiment, and would not be repeated here. The main difference between this embodiment and the thirty-third embodiment is the structure of the ablation catheter.

In this embodiment, the ablation catheter 3623 is an inner sheath 3632, and the distal end of the inner sheath 3622 is detachably connected to the support 3610. The inner sheath 3632 is rod-shaped or tubular, and the second conductive portion 3632 is provided on the outer peripheral wall of the inner sheath 3632. The second conductive portion 3632 may be an electrode or an exposed part of a conductive piece of the inner sheath 3632. The first conductive portion 3631 is provided on the proximal outer peripheral wall of the support 3610.

### Thirty-seventh embodiment

FIG. 40 is a schematic view of an ablation system 3700 according to the thirty-seventh embodiment of the present disclosure.

The ablation system 3700 in this embodiment is similar to the ablation system 3300 in the thirty-third embodiment. The similarities therebetween refer to the above description in the thirty-third embodiment, and would not be repeated here. The main difference between this embodiment and the thirty-third embodiment is the structure of the ablation catheter.

In this embodiment, the ablation catheter 3723 includes an outer sheath 3722 and a spherical mesh disposed at the distal end of the outer sheath 3722. The second conductive portion 3732 is at least one wave-like electrode disposed on the peripheral edge area of the spherical mesh, so that the second conductive portion 3732 can contact the tissue closely during the ablation. The spherical mesh can be made by weaving or cutting, and the spherical mesh is radially compressible and expandable. In the natural state without external force, the spherical mesh can be in a regular or irregular spherical shape, for example, in a sphere or an ellipsoid shape. The first conductive portion 3731 is provided on the proximal outer peripheral wall of the support 3710.

The delivery device 3720 has a double-layer sheath, with the inner sheath 3721 passing through the outer sheath 3722 and being configured as a delivery catheter to connect with the support. The distal end of the spherical mesh is connected to the distal end of the inner sheath 3721. Before the spherical mesh performs ablation, the diameter of the spherical mesh and the second conductive portion 3732 can be adjusted through the inner and outer sheaths. Specifically, the diameter of the spherical mesh and the second conductive portion 3732 can be adjusted by advancing the outer sheath 3722 distally, and/or pulling the inner sheath 3721. When the spherical mesh is compressed, the radial dimension of the second conductive portion 3732 is increased, so that its edge can contact the tissue outside the ostium of the left atrial appendage. After the ablation is completed, the axial length of the spherical mesh is increased by adjusting the inner and outer sheaths until the spherical mesh can be stretched for delivery.

It can be understood that in a natural state without external force, the spherical mesh can be in a regular closed shape, for example, in a sphere, an ellipsoid, or a column shape, or in an irregular closed shape.

### Thirty-eighth embodiment

FIG. 41 is a schematic view of an ablation system 3800 according to the thirty-eighth embodiment of the present disclosure.

The ablation system 3800 in this embodiment is similar to the ablation system 3700 in the thirty-seventh embodiment. The similarities therebetween refer to the above description in the thirty-seventh embodiment, and would not be repeated here. The main difference between this embodiment and the thirty-seventh embodiment is the structure of the ablation catheter.

In this embodiment, the ablation catheter 3823 is a balloon disposed on the delivery device 3820. After the balloon is filled with a medium, it is inflated outside the ostium of the left atrial appendage. The filled balloon is disc-shaped. The second conductive portion 3832 is provided on the outer peripheral edge of the filled balloon. After the ablation is completed, the balloon is recovered into the delivery device. The support 3810 is provided with a first conductive portion 3831 on the proximal outer peripheral surface thereof.

### Thirty-ninth embodiment

FIG. 42 is a schematic view of an ablation system 3900 according to the thirty-ninth embodiment of the present disclosure.

The ablation system 3900 in this embodiment is similar to the ablation system 3300 in the thirty-third embodiment. The similarities therebetween refer to the above description in the thirty-third embodiment, and would not be repeated here. The main difference between this embodiment and the thirty-third embodiment is the structure of the ablation catheter.

In this embodiment, the ablation catheter 3920 includes an outer sheath 3921 and a ring 3923 disposed at the distal end of the outer sheath 3921, and the second conductive portion 3932 is provided on the ring 3923. The ring 3923 is coiled around the axis of the outer sheath 3921 in a plane or a three-dimensional space, and the ring 3923 circles around the axis of the outer sheath 3921 at least once, for example, several times. The second conductive portion 3932 includes a plurality of portions arranged at intervals along the length direction of the ring 3923, such as a plurality of electrode elements arranged at intervals in sequence along the length direction of the ring 3923. Alternatively, the second conductive portion 3932 may be part or the entire of the ring 3923, and the second conductive portion 3932 may be distributed continuously or include portions distributed at intervals. The first conductive portion 3931 is provided on the proximal outer peripheral surface of the support 3910.

### Fortieth Embodiment

FIG. 43 is a schematic view of an ablation system 4000 according to the fortieth embodiment of the present disclosure.

The ablation system 4000 in this embodiment is similar to the ablation system 3300 in the thirty-third embodiment as shown in FIG. 35. The similarities therebetween refer to the above description in the thirty-third embodiment, and would not be repeated here. The main difference between this embodiment and the thirty-third embodiment is the structure of the ablation catheter.

In this embodiment, the ablation catheter 4023 includes an inner sheath 4021, an outer sheath 4022 and a plurality of support struts 4024. The inner sheath 4021 passes through the outer sheath 4022. The distal ends of the support struts 4024 are joined together and connected to the distal end of the inner sheath 4021, and the proximal ends of the support struts 4024 are joined together and connected to the distal end of the outer sheath 4022. The support strut 4024 is helical, facilitating the deformation during load and release. During the ablation, the radial dimension of the support struts 4024 is changed by pulling the inner sheath 4021 of the delivery device 4020 and/or pushing the outer sheath 4022 distally, and thus the support struts 4024 are compressed in the axial direction, and the radial dimension of the support struts 4024 is increased. The helical support struts 4024 can adapt to the morphology of the tissue at the ostium, and contact the tissue after deformation, without exerting great pressure on the tissue. The second conductive portion 4032 provided on the helical support strut 4024 is configured to ablate the tissue. The first conductive portion 4031 is provided on the proximal outer peripheral surface of the support 4010.

After the support struts 4024 are compressed to a certain extent in the radial dimension, the helical support struts 4024 tend to overlap each other in the axial direction to form a structure, i.e., a stable disc structure, for contacting the outer (proximal) tissue of the ostium. A plurality of electrode elements of the second conductive portion 4032 are disposed on each support strut 4024 at intervals. Preferably, the second conductive portion 4032 is at least disposed on the portion of the axially compressed support strut 4024 with a large radial dimension, facilitating the ablation on the tissue in the edge area outside the ostium. After the ablation is completed, the support struts 4024 can be recovered in the outer sheath 4022. In some embodiments, the support struts 4024 may have no helix angle.

### Forty-first embodiment

FIG. 44 is a schematic view of an ablation system 4100 according to the forty-first embodiment of the present disclosure.

The ablation system 4100 in this embodiment is similar to the ablation system 4000 in the fortieth embodiment. The similarities therebetween refer to the above description in the fortieth embodiment, and would not be repeated here. The main difference between this embodiment and the fortieth embodiment is the structure of the support.

In this embodiment, the support 4110 has a single-disc columnar structure, which can be made by weaving or cutting. The first conductive portion 4131 is disposed on the proximal outer periphery of the support 4110, and the first conductive portion 4131 is a ring electrode. The second conductive portion 4132 is provided on the delivery device 4120. The first conductive portion 4131 and the second conductive portion 4132 cooperate with each other to perform ablation.

### Forty-second embodiment

FIG. 45 is a schematic view of an ablation system 4200 according to the forty-second embodiment of the present disclosure, and FIG. 46 is a schematic view showing the ablation system ablating the tissue of the left atrial appendage.

The ablation system 4200 in this embodiment is similar to the ablation system 3300 in the thirty-third embodiment shown in FIG. 35. The similarities therebetween refer to the above description in the thirty-third embodiment, and would not be repeated here. The main difference between this embodiment and the thirty-third embodiment is that the structure of the support and the arrangement of the delivery device are different.

Referring to FIG. 45 and FIG. 46, in this embodiment, the second conductive portion 4232 is provided on the delivery device, and the second conductive portion 4323 is movable relative to the support 4210 and configured to be released on the distal side of the support 4210. The support 4210 is a double-disc structure. The structure of the support 4210 is similar to that shown in FIG. 21 and FIG. 22. The support 4210 includes a sealing part 4211 and an anchoring part 4212, which are connected by a connecting piece 4217 and electrically isolated from each other. In the space enclosed by the support 4210, a channel is defined in the axial direction. The distal end of the ablation catheter 4223 passes through the channel and can be released on the distal side of the anchoring part 4212.

The sealing part 4211 is trapezoidal, including a distal disc surface 4218 facing the anchoring part 4212, a proximal disc surface 4219 facing away from the anchoring part 4212, and a waist 4220 between the distal disc surface 4218 and the proximal disc surface 4219. The proximal disc surface 4219 and the distal disc surface 4218 are generally planar, and the waist 4220 is connected between the proximal disc 4219 and the distal disc 4218 and is in the shape of a cone.

The first conductive portion 4231 is provided at least in the edge area of the sealing part 4211 with a large radial dimension, such as on the waist 4220. Preferably, the first conductive portion 4231 is provided on a portion of the sealing part 4211 with the maximum radial dimension in the circumferential direction. Alternatively, the first conductive portion 4231 may be provided on the waist 4220 and the proximal disc surface 4219, or be provided on at least part of the waist 4220, the proximal disc surface 4219 and the distal disc surface 4218. The first conductive portion 4231 is part of the first frame of the sealing part 4211. The surface of the sealing part 4211 that is not used as the first conductive portion 4231 can be subjected to insulation treatment.

Specifically, the main difference between the sealing part 4211 and the sealing parts as shown in FIG. 21 and FIG. 22 is the mesh. The mesh size of the proximal portion of the sealing part 4211 is different from that of the distal portion of the sealing part 4211. Specifically, the meshes of the proximal portion of the sealing part 4211 are smaller and more in number, while the meshes of the distal portion of the sealing part 4211 are larger and less in number. The first frame with the small meshes of the sealing part 4211 is used as the first conductive portion, and the surface of the first frame with the large meshes of the sealing part 4211 is subjected to insulation treatment. Specifically, the portion with the small meshes is formed by weaving wires into a dense mesh, and the portion with the large meshes is formed by extending multi-strand wires of the portion with the small meshes into bundles, in which the multi-strand wires in each bundle are fixed to each other. There are no intersections with mutual friction in the first frame of the portion with the large meshes, or the density of the intersections with mutual friction in the first frame of the portion with the large meshes (for example, there may be mutual friction between two bundles of wires) is relatively lower than the portion with the small meshes, to facilitate insulation treatment on the first frame of the portion with the large meshes.

The anchoring part 4212 is disc-shaped similar to that shown in FIG. 21 and FIG. 22. The anchoring part 4212 includes a plurality of anchoring struts, including first anchoring struts 4213, second anchoring struts 4214, third anchoring struts 4215 and fourth anchoring struts 4216 connected in sequence. The proximal end of the first anchoring strut 4213 is connected to the insulating connecting piece, and the first anchoring strut 4213 extends between the proximal end and the distal end. The ends of two adjacent second anchoring struts 4214 away from the first anchoring struts 4213 are connected. The third anchoring strut 4215 extends between the proximal end and the distal end, and is located on the outer peripheral side of the anchoring part 4212, for contacting the inner wall of the left atrial appendage. An end of the fourth anchoring strut 4216 away from the third anchoring strut 4215 extends toward the axis and the distal side. The ends of two adjacent fourth anchoring struts 4216 away from the third anchoring struts 4215 are connected together to improve the mechanical properties of the anchoring part 4212 and prevent the fourth anchoring struts 4212 from interlocking with each other during the radial deformation of the anchoring part 4212, thereby improving the reliability of the support 4210. Each third anchoring strut 4215 is provided with two dot-shaped electrodes spaced apart from each other.

Similar to the ablation catheter as shown in FIG. 35, the ablation catheter 4223 has an umbrella-shaped frame. The ablation catheter 4223 includes a plurality of support struts 4224 arranged in a radial pattern around the axis of the ablation catheter, and an electrode element is provided at the end of each support strut 4224, thus forming the second conductive portion 4232 at the end of the umbrella-shaped frame.

The ablation catheter 4223 in this embodiment differs from the ablation catheter in FIG. 35 in that the ablation catheter 4223 is configured to be released on the distal side of the support 4210.

Specifically, referring to FIG. 45 and FIG. 46, the support 4210 is delivered to the ostium of the left atrial appendage through the delivery device 4240. The waist 4220 of the sealing part 4211 contacts the ostium of the left atrial appendage, the first conductive portion 4231 on the waist 4220 contacts the proximal tissue of the left atrial appendage, and the third anchoring struts 4215 contact the inner wall of the left atrial appendage. When the pulse ablation source is powered on, the shortest electric field line E between the first conductive portion 4231 and the second conductive portion 4232 passes through the tissue at the ostium, which facilitates transmural ablation at the ostium. After the ablation is completed, the ablation catheter 4223 is withdrawn. The distal end of the ablation catheter 4223 in this embodiment passes through the axial channel of the support 4210, and can be released on the distal side of the anchoring part 4212.

In other embodiments, the support uses the structures according to other embodiments, and the release position of the second conductive portion on the delivery device can be adjusted according to the specific structure of the support. For example, in the case where the anchoring part of the support is cup-shaped, and the distal end is opened, then the second conductive portion can be released on the distal side of the support. Specifically, the second conductive portion can be released in the anchoring part, at the distal end of the support or beyond the distal end of the support.

### Forty-third embodiment

FIG. 47 is a schematic view of an ablation system 4300 according to the forty-third embodiment of the present disclosure.

The ablation system 4300 in this embodiment is similar to the ablation system 4200 in the forty-second embodiment. The structures of the supports therebetween are the same. The similarities therebetween refer to the above description in the forty-second embodiment, and would not be repeated here. The main difference between this embodiment and the forty-second embodiment is the structure of the ablation catheter.

In this embodiment, the structure of the ablation catheter 4323 is the same as that of the ablation catheter in the thirty-fourth embodiment as shown in FIG. 37. The second conductive portion 4332 is provided on the support rings, which will not be repeated here. Different from the ablation system 3400 of the thirty-fourth embodiment, the distal end of the ablation catheter 4323 in this embodiment passes through the axial channel of the support 4300 and can be released on the distal side of the anchoring part 4312. By contrast, the ablation catheter in the thirty-fourth embodiment is released on the proximal side of the sealing part.

### Forty-fourth embodiment

FIG. 48 is a schematic view of an ablation system 4400 according to a forty-fourth embodiment of the present disclosure.

The ablation system 4400 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the ablation catheter.

In this embodiment, the structure of the ablation catheter 4423 is the same as that of the ablation catheter 3523 in the thirty-fifth embodiment as shown in FIG. 38 and also has a meshed disc structure. The second conductive portion 4432 is provided on the meshed disc structure. The first conductive portion 4431 is provided on the sealing part 4411. Different from the ablation system in the thirty-fifth embodiment, the distal end of the ablation catheter 4423 in this embodiment passes through the axial channel of the support 4410 and can be released on the distal side of the anchoring part 4412. By contrast, the ablation catheter of the thirty-fifth embodiment is released on the proximal side of the sealing part.

### Forty-fifth embodiment

FIG. 49 is a schematic view of an ablation system 4500 according to the forty-fifth embodiment of the present disclosure.

The ablation system 4500 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the ablation catheter.

In this embodiment, in the delivery state, the ablation catheter 4523 is linearly accommodated in the outer catheter of the delivery device. After the ablation catheter 4523 is released from the outer catheter, the end of the ablation catheter 4523 away from the support 4510 bends and extends radially outwardly, and coils into a ring. In some embodiments, the ablation catheter 4523 is linear after being released from the outer catheter, and the second conductive portion 4532 is provided on the side wall of the delivery catheter. The first conductive portion 4531 is provided on the sealing part 4511. The second conductive portion 4532 may be an electrode or an exposed part of a conductive piece of the delivery catheter.

### Forty-sixth embodiment

FIG. 50 is a schematic view of an ablation system 4600 according to the forty-sixth embodiment of the present disclosure.

The ablation system 4600 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the ablation catheter.

In this embodiment, the first conductive portion 4631 is provided on the sealing part 4611. The ablation catheter 4623 is a spherical mesh, and the second conductive portion 4632 is provided on the peripheral edge area of the spherical mesh. The spherical mesh is the same as the spherical mesh of the thirty-seventh embodiment as shown in FIG. 40, and will not be repeated here. Different from the ablation system in the thirty-seventh embodiment, the distal end of the ablation catheter 4623 in this embodiment passes through the axial channel of the support 4610 and can be released on the distal side of the anchoring part. By contrast, the ablation catheter of the thirty-seventh embodiment is released on the proximal side of the sealing part.

### Forty-seventh embodiment

FIG. 51 is a schematic view of an ablation system 4700 according to the forty-seventh embodiment of the present disclosure.

The ablation system 4700 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the ablation catheter.

In this embodiment, the ablation catheter 4723 is a balloon, which is similar to the ablation catheter according to the thirty-eighth embodiment as shown in FIG. 41. After the balloon is filled with a medium, it is inflated inside the left atrial appendage. The inflated balloon has a disc shape, and the second conductive portion 4732 is provided on the peripheral edge of the inflated balloon. After the ablation is completed, the balloon is recovered into the outer sheath. The first conductive portion 4731 is provided on the sealing part 4711. The distal end of the ablation catheter 4723 in this embodiment passes through the axial channel of the support 4710 and can be released on the distal side of the anchoring part 4712. By contrast, the ablation catheter of the thirty-eighth embodiment is released on the proximal side of the sealing part.

### Forty-eighth embodiment

FIG. 52 is a schematic view of an ablation system 4800 according to the forty-eighth embodiment of the present disclosure.

The ablation system 4800 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the ablation catheter.

In this embodiment, the ablation catheter 4823 is a ring, which is similar to the ablation catheter in the thirty-ninth embodiment as shown in FIG. 42. The second conductive portion 4832 is provided on the ring, and the first conductive portion 4831 is provided on the sealing part 4811. The second conductive portion 4832 includes a plurality of portions arranged at intervals along the length direction of the ring, such as a plurality of electrode elements arranged at intervals in sequence along the length direction of the ring. Alternatively, the second conductive portion 4832 may be part or the entire of the ring, and the second conductive portion 4832 can be distributed continuously or the second conductive portion 4832 can include portions distributed at intervals. The distal end of the ablation catheter 4823 in this embodiment passes through the axial channel of the support 4810 and can be released on the distal side of the anchoring part 4812. By contrast, the ablation catheter of the thirty-ninth embodiment is released on the proximal side of the sealing part.

### Forty-ninth embodiment

FIG. 53 is a schematic view of an ablation system 4900 according to the forty-ninth embodiment of the present disclosure.

The ablation system 4900 in this embodiment is similar to the ablation system 4200 in the forty-second embodiment as shown in FIG. 45. The similarities therebetween refer to the above description in the forty-second embodiment, and would not be repeated here. The main difference between this embodiment and the forty-second embodiment is the structure of the support and the arrangement of the first conductive portion.

In this embodiment, the support 4910 is a double-disk structure. The second conductive portion 4932 is provided on the delivery device, and the second conductive portion 4932 is movable relative to the support 4910 and configured to be released on the distal side of the support 4910. The first conductive portion 4931 is disposed adjacent to the proximal end of the support 4910. The support 4910 includes a sealing part 4911 and an anchoring part 4912 which are insulated from and connected with each other by a connecting piece 4913. The sealing part 4911 has a disc-shaped structure with a short axial length. The anchoring part 4912 is cup-shaped with an open distal end. The anchoring part 4912 can be made by weaving wires or by cutting a tube. The proximal ends of the wires of the anchoring part 4912 are converged in the connecting piece 4913 radially inwardly. The first conductive portion 4931 is disposed on the outer peripheral surface of the anchoring part 4912. The first conductive portion 4931 is a ring electrode.

The ablation catheter 4923 in this embodiment has the same structure as the ablation catheter 4223 in the forty-second embodiment. The ablation catheter 4923 also includes an umbrella-shaped frame including a plurality of support struts 4924 arranged in a radial pattern around its axis. An electrode element is provided at the end of each support strut 4924, thus forming the second conductive portion 4932 at the end of the umbrella-shaped frame. Since the distal end of the anchoring part 4912 is open, the ablation catheter 4923 can be released inside the anchoring part 4912.

### Fiftieth Embodiment

FIG. 54 is a schematic view of an ablation system 5000 according to a fiftieth embodiment of the present disclosure.

The structure of the ablation system in this embodiment is similar to that of the ablation system 4900 in the forty-ninth embodiment as shown in FIG. 53. The similarities therebetween refer to the above description in the forty-ninth embodiment, and would not be repeated here. The main difference between this embodiment and the forty-ninth embodiment is the structure of the ablation catheter.

In this embodiment, the second conductive portion 5032 is provided on the delivery device, and the second conductive portion 5032 is movable relative to the support 5023 and configured to be released inside the support 5023. The anchoring part 5012 of the support 5023 is cup-shaped and has an open distal end, and the second conductive element 5032 can be released in the anchoring part 5012. The ablation catheter 5023 is a balloon. After the balloon is filled with a medium, it is inflated inside the ostium of the left atrial appendage, and the filled balloon is disc-shaped. The support 5010 includes a sealing part 5011 and an anchoring part 5012 connected to each other. The first conductive portion 5031 is a ring electrode fixed on the outer peripheral wall of the anchoring part 5012. The second conductive portion 5032 is provided on the outer peripheral edge of the inflated balloon. The balloon is recovered into the outer sheath 5022 after the ablation is completed. Since the distal side of the anchoring part 5012 is open, the balloon can be released inside the anchoring part 5012.

It can be understood that, for various supports 5010 having an anchoring part 5012 with an open distal end, for example, a columnar or cup-shaped anchoring part 5012, the respective ablation catheter 5023 can be released inside the anchoring part 5012. In the forty-ninth embodiment and the fiftieth embodiment, the ablation catheter 5023 can be released inside the respective anchoring part 5012. The second conductive portion 5032 can be enlarged in the radial dimension to contact the tissue by passing through the meshes of the anchoring part 5012. Alternatively, the second conductive portion 5032 can perform ablation on the tissue of the left atrial appendage by contacting the blood without being deformed to increase the radial dimension.

### Fifty-first embodiment

FIG. 55 is a schematic view of an ablation system 5100 according to the fifty-first embodiment of the present disclosure.

In this embodiment, the support 5110 has a double-disc structure, and the first conductive portion 5131 and the second conductive portion 5132 are both provided on the support 5110.

The support 5110 includes a sealing part 5111 and an anchoring part 5112. The sealing part 5111 includes a first frame 5113, and the anchoring part 5112 includes a second frame 5117. The second frame 5117 of the anchoring part 5112 is made of metal wires by weaving, and the woven second frame 5117 includes inner and outer layers. Specifically, the metal wires extend radially from the proximal end to the distal end of the anchoring part 5112 to form the inner layer of the second frame 5117, and then turn back from the distal end to the proximal end and are woven to form the outer layer of the second frame 5117.

The second conductive portion is disposed on the second frame 5117 of the anchoring part 5112. In this embodiment, the second conductive portion is a second electrode element disposed on the second frame 5117, and the second electrode element is configured as the second conductive portion 5132. The surface of the second frame 5117 that is not connected to the second electrode element is insulated, that is, the anchoring part 5112 is designed such that only the surface of the second frame 5117 that does not correspond to the second electrode element is insulated.

Preferably, the second conductive portion 5132 uses a ring electrode, that is, the second electrode element is a ring electrode, and the ring electrode is fixed on the outer peripheral wall of the anchoring part 5112. The anchoring part 5112 is designed such that the entire surface of the second frame 5117 is insulated, and the ring electrode is an electrode additionally disposed thereon. Specifically, the surface insulation of the second frame 5117 can be realized by providing an insulating film on the second frame 5117. The part of the second frame 5117 corresponding to the second electrode element is the part where the second frame 5117 is in contact with the second electrode element. Accordingly, the part of the second frame 5117 not corresponding to the second electrode element is the part where the second frame 5117 is not in contact with the second electrode element. It can be understood that, in some other embodiments, the second electrode element may use arrangements that extends in the circumferential direction in a curved course, such as in a zigzag shape, a wave shape, and the like.

Specifically, the first frame 5113 is a structure made from metal wire by weaving. The sealing part 5111 includes a disc surface 5114 facing away from the anchoring part 5112, a disc bottom 5115 facing the anchoring part 5112, and a waist 5116 connected between the disc surface 5114 and the disc bottom 5115. The diameter of the disc surface 5114 is greater than the diameter of the disc bottom 5115, and the diameter of the disc surface 5114 is slightly greater than the inner diameter of the left atrial appendage. After the support 5110 is implanted into the left atrial appendage, the disc surface 5114 contacts the ostium of the left atrial appendage, and the disc bottom 5115 is inserted into the left atrial appendage. The diameter of the disc bottom 5115 is generally the same as the inner diameter of the left atrial appendage. A first electrode element is provided on the waist 5116 of the sealing part 5111 as the first conductive portion 5131, to contact the ostium of the left atrial appendage.

In the support 5110 shown in this embodiment, the first conductive portion 5131 on the sealing part 5111 is part of the first frame 5113, and the second conductive portion 5132 on the anchoring part 5112 is a second electrode element disposed on the second frame. It can be understood that, the frame or an additional electrode element can be selected as the conductive portion as required.

### Fifty-second embodiment

FIG. 56 is a schematic view of an ablation system 5200 according to the fifty-second embodiment of the present disclosure.

The structure of the support 5210 of the ablation system 5200 in this embodiment is similar to the structure of the support 5110 of the fifty-first embodiment as shown in FIG. 55. The similarities therebetween refer to the above description in the fifty-first embodiment, and would not be repeated here. The main difference between this embodiment and the fifty-first embodiment is the arrangement of the second conductive portion.

In this embodiment, the support 5210 is provided with two first conductive portions 5231 spaced apart from each other in the axial direction, which are respectively provided on the sealing part 5211 and the anchoring part 5212. The first conductive portion 5231 provided on the sealing part 5211 is at least part of the first frame, and the first conductive portion 5231 on the anchoring part 5212 is a ring electrode fixed on the outer wall. The second conductive portion 5232 is provided on the delivery device 5220.

The delivery device 5220 includes a delivery catheter, and the second conductive portion 5232 is a meshed disc structure provided on the delivery catheter, which is similar to the structure of the ablation catheter in the thirty-fifth embodiment as shown in FIG. 38. Preferably, the entire meshed disc is configured as the second conductive portion 5232, so that the entire woven meshed disc can be used as an electrode to transmit ablation energy to the tissue. The distal end of the delivery catheter in this embodiment passes through the axial channel of the support 5210 and can be released on the distal side of the anchoring part 5212.

In some embodiments, the anchoring part can be subjected to insulation treatment. For example, the support frame of the anchoring part can be provided with an insulating coating or an insulating sleeve, or the anchoring part can be made of insulating material. By subjecting the anchoring part to insulation treatment, the anchoring part and the second conductive portion can be prevented from being in contact with each other to conduct electricity, and thus the problem of an increased discharge area of the second conductive portion can be avoided, to improve the stability of the system and the success rate of ablation.

### Fifty-third embodiment

FIG. 57 is a schematic view of an ablation system 5300 according to the fifty-third embodiment of the present disclosure.

The ablation system 5300 of this embodiment is configured to ablate the pulmonary vein, atrium, left atrial appendage in the heart, or tissues to be ablated outside the heart. The support is a radially compressible and expandable frame, and the delivery device is a delivery catheter. The ablation system 5300 includes a distal frame 5310 and a proximal delivery catheter 5320. The frame 5310 is connected to the distal end of the delivery catheter 5320, and is configured to be delivered to the vicinity of the tissue to be ablated along the delivery path and released there to realize tissue ablation. The radial dimension of the frame 5310 can be adjusted through the delivery catheter 5320, thereby realizing radial compression and expansion. Specifically, the radial dimension of the frame 5310 can be adjusted by moving the outer sheath 5322 and the inner sheath 5321 of the delivery catheter 5320 relative to each other, for example, by pulling the inner sheath 5321 proximally and/or pushing the outer sheath 5322 distally to cause relative movement between the outer sheath 5322 and the inner sheath 5321.

In this embodiment, the support uses a diameter-varying frame 5310. That is, the expanded frame 5310 has a large-diameter end close to the proximal end and a small-diameter end close to the distal end, and the maximum radial dimension of the large-diameter end is greater than the maximum radial dimension of the small-diameter end. The axial length of the large-diameter end is greater than the axial length of the small-diameter end. The frame 5310 includes an accommodating part 5330 at the junction of the large-diameter end and the small-diameter end. The accommodating part 5330 is arranged adjacent to the axis of the frame 5310 relative to the first conductive portion 5331 and/or the second conductive portion 5332. In this embodiment, the accommodating part 5330 is arranged adjacent to the axis of the frame 5310 relative to the first conductive portion 5331 and the second conductive portion 5332. That is, a recess is formed on the frame 5310, and an accommodating space is defined outside the outer wall of the accommodating part 5330 for accommodating tissue to be ablated, which facilitates the shortest electric field line E to pass through the tissue located in the accommodating space. Preferably, at least one shortest electric field line E passing through the accommodating space is inclined relative to the axis of the frame 5310, so that the accommodating part 5330 of the frame 5310 can accommodate more tissue, and the shortest electric field line E can pass through the tissue in the accommodating space more easily. In this embodiment, the first conductive portion 5331 is disposed on the distal side of the large-diameter end, and the second conductive portion 5332 is disposed on the proximal side of the small-diameter end. The radial dimension of the first conductive portion 5331 is greater than the radial dimension of the second conductive portion 5332, that is, the radial dimension of the ring structure formed by the proximal conductive portion is greater than the radial dimension of the ring structure formed by the distal conductive portion, so that at least one shortest electric field line E passing through the accommodating space is inclined relative to the axis of the frame 5310, facilitating the shortest electric field line to pass through the tissue in the accommodating space.

It can be understood that the frame 5310 can use other diameter-varying structures. For example, the maximum radial dimension of the proximal end and the maximum radial dimension of the distal end can be equal, an accommodating part is formed between the proximal end and the distal end, and the radial dimension of the first conductive portion 5331 is different from the radial dimension of the second conductive portion 5332 so that at least one shortest electric field line E passing through the accommodating space is also inclined relative to the axis of the frame 5310, and thus also facilitating the shortest electric field line to pass through the tissue in the accommodating space.

As shown in FIG. 57, the accommodating part 5330 is arranged adjacent to the axis of the frame 5310 relative to the first conductive portion 5331 and the second conductive portion 5332. The electric field lines passing through the accommodating space are denser, the electric field intensity is greater, and the tissue in the accommodating space is more likely to be ablated. The first conductive portion 5331 and the second conductive portion 5332 are provided on opposite sides of the accommodating space, and the radial dimension of the first conductive portion 5331 is greater than the radial dimension of the second conductive portion 5332, so that at least one shortest electric field line E passing through the accommodating space is inclined relative to the axis of the frame 5310.

Specifically, the frame 5310 includes a plurality of support struts 5311 arranged at intervals in the circumferential direction around the axis of the frame 5310, and the number of the support struts 5311 may specifically be 4, 6, 8 or more. The distal ends of the support struts 5311 are joined together, and the proximal ends are joined together. Each support strut 5311 includes a first arc segment 5312, a second arc segment 5313 and a third arc segment 5314 connected in sequence. When the frame 5310 is expanded, both the first arc segments 5312 and the third arc segments 5314 bend away from the central axis of the frame, while the second arc segments 5313 bends toward the central axis of the frame, so that the accommodating part 5330 is formed at the second arc segments 5313 of the frame 5310. The radial dimension of the first arc segment 5312 is greater than the radial dimension of the third arc segment 5314, and the axial dimension of the first arc segment 5312 is greater than the axial dimension of the third arc segment 5314. In other embodiments, the axial dimension of the first arc segment 5312 is not greater than the axial dimension of the third arc segment 5314.

The first conductive portion 5331 is provided at the junctions of the first arc segments 5312 and the second arc segments 5313, and the second conductive portion 5332 is provided at the junctions of the second arc segments 5313 and the third arc segments 5314. That is, the first conductive portion 5331 is located at the proximal side of the accommodating part 5330, and the second conductive portion 5332 is located at the distal side of the accommodating part 5330. When the frame 5310 is expanded, the radial dimension of the first conductive portion 5331 is greater than the radial dimension of the second conductive portion 5332. When the delivery catheter 5320 deliveries the frame 5310 to the target area, the accommodating part 5330 contact the ostium of the pulmonary vein or the left atrial appendage, and the shortest electric field line E can pass through the tissue.

As shown in FIG. 57, each support strut 5311 is surrounded by two ring-shaped electrodes at different axial levels, so that two rings of electrodes spaced in the axial direction are formed on the frame 5310. The first conductive portion 5313 includes a ring of electrodes spaced apart from each other in the circumferential direction, and the second conductive portion includes another ring of electrodes spaced apart from each other in the circumferential direction.

In this embodiment, the conductive portions on two sides of the accommodating part 5330 are spaced apart in the axial direction. The accommodating part 5330 can extend in the circumferential direction and the axial direction, or in an irregular direction. The conductive portions on two sides are disposed on opposite sides of the accommodating part 5330, so that the shortest electric field line E can pass through the area outside the frame 5310, and thus can easily pass through the tissue during the ablation, achieving transmural ablation.

In some other embodiments, the frame 5310 can be replaced with a balloon, such as a balloon made by cutting or weaving which is compressible and expandable in the radial direction. The balloon is filled with a medium, and the radial size of the balloon can adjusted by charging or discharging the medium into or out from the balloon.

### Fifty-fourth Embodiment

FIG. 58 is a schematic view of an ablation system 5400 according to the fifty-fourth embodiment of the present disclosure.

In this embodiment, both the first conductive portion 5431 and the second conductive portion 5432 are provided on the support 5410, that is, the first conductive portion 5431 and the second conductive portion 5432 are provided on the support 5410 at an interval in the axial direction. The support 5410 has a structure with a large proximal end and a small distal end, that is, the radial dimension of the proximal end of the support 5410 is greater than the radial dimension of the distal end. The frame is provided with an accommodating part at the portion between the first conductive portion 5431 and the second conductive portion 5432. It can be understood that, for supports with different shapes and structures, the accommodating parts formed thereon and the respective accommodating spaces outside are different. In this embodiment, the accommodating part is formed between the sealing part and the anchoring part.

Specifically, the first conductive portion 5431 is provided on the proximal side of the support 5410, specifically on the sealing part. The second conductive portion 5432 is located on the distal side of the first conductive portion 5431, specifically on the anchoring part. The radial dimensions of the portions of the support 5410 where the two conductive portions are provided are different, and the radial dimension of the first conductive portion 5431 is greater than that of the second conductive portion 5432. When the support 5410 is delivered to the ostium of the left atrial appendage, the shortest electric field line E between the first conductive portion 5431 and the second conductive portion 5432 can easily pass through the area outside the accommodating part of the support 5410, and the support 5410 between the two conductive portions is configured to contact the tissue, which facilitates the shortest electric field line to pass through the tissue during the ablation.

The support 5410 includes a sealing part 5411 and an anchoring part 5412 connected to each other, and the anchoring part 5412 is provided with anchoring barbs 5413. In the case where the anchoring barbs 5413 are removed, the ablation catheter can be used to perform ablation on sites such as the pulmonary vein and the left atrial appendage. It can be understood that part of the first conductive portion 5431 may only be used for mapping and cannot be used for ablation. In some embodiments, part of the first conductive portion 5431 may be used for mapping, and the other may be used for ablation. In some embodiments, at least part of the first conductive portion 5431 may be used for mapping and ablation, respectively, in different periods.

Specifically, the support 5410 includes a frame 5414. The frame 5414 may have a blocking piece(s) for blocking thrombus, and the number and location of the blocking piece are not limited. For illustration, the blocking piece is not shown in FIG. 58. The frame 5414 includes a first frame 5415, a second frame 5416 and an insulating part 5417. Both the first frame 5415 and the second frame 5416 are made of conductive material, and the first frame 5415 and the second frame 5416 are connected by the insulating part 5417. The first frame 5415 and the second frame 5416 are respectively configured to transmit ablation energy with different polarities to the target tissue area.

Both the first frame 5415 and the second frame 5416 can be made of metal material or polymer material with good biocompatibility. The metal material can be nickel-titanium alloy, cobalt-chromium alloy, stainless steel and degradable metal material, preferably superelastic shape memory alloy of nickel-titanium wire. The insulating part is made of insulating material with good biocompatibility. In some embodiments, the first frame 5415 and the second frame 5416 are made by weaving. In some embodiments, one of the first frame 5415 and the second frame 5416 is made by weaving, and the other is made by cutting.

The frame 5414 can be made by preparing the first frame 5415 and the second frame 5416 by cutting conductive tubes respectively, and connecting the insulating part 5417 with the first frame 5415 and the second frame 5416 therebetween by one or more of welding, plug-in connection and snap-fit.

As shown in FIG. 58, the first frame 5415 and the second frame 5416 are axially spaced apart, and the insulating part 5417 defines a ring-like insulating area around the axis of the support 5410. The ablation element 5430 includes a first conductive portion 5431 provided on the first frame 5415 and a second conductive portion 5432 provided on the second frame 5416. In this embodiment, the first conductive portion 5431 is at least part of the first frame 5415, and the second conductive portion 5432 is at least part of the second frame 5416. The surface of the first frame 5415 other than the first conductive portion 5431 is subjected to insulation treatment. The surface of the second frame 5416 other than the second conductive portion 5432 is subjected to insulation treatment. That is, both the first frame 5415 and the second frame 5416 ablate the target tissue area through parts of the surfaces of the support struts.

In this embodiment, both the first frame 5415 and the second frame 5416 are made by cutting and have a plurality of meshes. The insulating part 5417 is connected between the first frame 5415 and the second frame 5416 and includes straight rods. In a modified embodiment, each support strut of the insulating part 5417 is in the shape of an arc, a fold line, or a ring. Alternatively, the insulating part 5417 has a larger axial size than that shown in FIG. 58. Alternatively, the plurality of support struts of the insulating part 5417 are connected to each other to form meshes. The specific form of the insulating part 5417 is not limited here. In a modified embodiment, at least one of the first frame 5415 and the second frame 5416 can be made by weaving.

In this embodiment, both the first conductive portion 5431 and the second conductive portion 5432 are parts of the frames. In a modified embodiment, at least one of the first conductive portion 5431 and the second conductive portion 5432 is an ablation electrode additionally disposed on the frame (the first frame 5415 or the second frame 5416), and the ablation electrode may be electrically connected with the respective frame, or be insulated from the frame and connected to an external ablation signal source through a conducting wire.

As shown in FIG. 58, the first frame 5415 and the second frame 5416 are axially spaced apart. In a modified embodiment, the first frame 5415 and the second frame 5416 are spaced apart in the circumferential direction. For example, the first frame 5415 can be arranged within a first angle range in the circumferential direction of the support 5410, the second frame 5416 can be arranged within a second angle range in the circumferential direction of the support 5410, and the first frame 5415 and the second frame 5416 are connected through an insulating part 5417.

In a modified embodiment, the first frame 5415 and the second frame 5416 are spaced apart in the circumferential direction and the axial direction.

The sealing part 5411 is configured to cover the ostium of the left atrial appendage, which is easier to cover the ostium of the left atrial appendage in a circle, so the first conductive portion 5431 provided on the sealing part 5411 facilitates to form a complete ring-like ablation zone at the ostium of the left atrial appendage, improving the ablation Effect. Preferably, the first conductive portion 5431 is provided on the peripheral edge area of the sealing part 5411. The first conductive portion 5431 can be provided on at least one of the proximal edge, the edge of the middle section, and the distal edge of the sealing part 5411. The first conductive portion 5431 is provided on the portion of the sealing part 5411 that can contact the ostium of the left atrial appendage in a circle. The portions on the sealing part 5411 have fixed distances to the axis of the sealing part 5411. In some embodiments, the peripheral edge area refers to the area on the sealing part 5411 that is 20% of the maximum distance to the axis of the sealing part 5411. In some embodiments, the peripheral edge area refers to the area on the sealing part 5411 that is 10% of the maximum distance to the axis of the sealing part 5411. In some embodiments, the peripheral edge area refers to the area on the sealing part 5411 that is 5% of the maximum distance to the axis of the sealing part 5411. In some embodiments, the peripheral edge area refers to the area on the sealing part 5411 that is 3% of the maximum distance to the axis of the sealing part 5411. It can be understood that the specific edge area can be determined in practice, and is not limited here.

In a modified embodiment, at least one of the first conductive portion 5431 and the second conductive portion 5432 is an ablation electrode. In the first conductive portion 5431 and the second conductive portion 5432, more ablation electrodes can be provided at intervals, and the respective locations of ablation electrodes are not limited here.

It should be noted that the specific technical features in the above embodiments can be replaced with each other without departing from the concept of the present invention. For example, on the basis of the above embodiments, the first conductive portion and the second conductive portion can be selected from a conductive frame, or additional dot-shaped electrodes, rod electrodes, arc electrodes, ring electrodes, wave-like electrodes, etc.; or the second conductive portion can be provided on the sealing part, the anchoring part, the connecting piece, the delivery device, or a component other than the support and the delivery device; or more above-mentioned first conductive portions and second conductive portions can be provided on the support; or the sealing parts, the anchoring parts, and the ablation catheters in the above embodiments can be combined to obtain a new ablation system; which all belong to the present invention. The first conductive portion and the second conductive portion in each of the above embodiments can adopt the parameters of electric field intensity, voltage, distance, and area provided by the embodiments of the present disclosure, and the first ablation zone and the second ablation zone partially overlapping each other can be applied to each of the above embodiments. In each of the above embodiments, the support can be provided with the accommodating part for accommodating tissue and provided between two conductive portions, at least one shortest electric field line passes through the accommodating space outside the accommodating part, and at least one shortest electric field line passing through the accommodating space is inclined relative to the axis of the support. Being inclined relative to the axis of the support means that at least one shortest electric field line passing through the accommodating space is neither parallel nor perpendicular to the axis of the support. As shown in the figures of the above embodiments, two included angles are defined between at least one shortest electric field line and the axis of the support, the sum of the two included angles is 180 degrees, and the smaller included angle is an acute angle.

The above are only preferred embodiments of the invention, and are not intended to limit the invention. Any modifications, alternatives and developments made within the spirit and concept of the invention should fall into the protection scope of the invention.

## Claims

1. An ablation system, comprising:
a support;
a delivery device, a distal end of the delivery device is connected to the support, and the delivery device is configured to deliver the support to a target tissue; and
an ablation element, comprising a first conductive portion and a second conductive portion, the first conductive portion provided on the support, the second conductive portion provided on the support or the delivery device or independently from the support and the delivery device, both the first conductive portion and the second conductive portion electrically connected to an external pulse ablation source, and the first conductive portion and the second conductive portion are configured to transmit pulse ablation energy with different polarities to the target tissue to achieve tissue ablation.

2. The ablation system according to claim 1, wherein during ablation, an intensity of an electric field generated by the ablation element at a distance of 2 mm from a surface of the ablation element is greater than 400 V/cm.

3. The ablation system according to claim 2, wherein during ablation, an intensity of an electric field generated by the ablation element at a distance of 3 mm from a surface of the ablation element is greater than 400 V/cm.

4. The ablation system according to any one of claims 1 to 3, wherein a pulse voltage for the ablation element is in a range of 500 V to 4000 V, a discharge area of the first conductive portion is in a range of 15 mm² to 4700 mm², a discharge area of the second conductive portion is in a range of 15 mm² to 4700 mm², and a distance between two closest points on surfaces of the first conductive portion and the second conductive portion is in a range of 1 mm to 45 mm.

5. The ablation system according to claim 4, wherein the distance between the two closest points on the surfaces of the first conductive portion and the second conductive portion is in a range of 3 mm to 22 mm.

6. The ablation system according to claim 5, wherein a zone where an intensity of an electric field generated around the first conductive portion greater than a field intensity threshold of the target tissue is a first ablation zone, a zone where an intensity of an electric field generated around the second conductive portion greater than the field intensity threshold of the target tissue is a second ablation zone, and the first ablation zone and the second ablation zone are partially overlapped with each other.

7. The ablation system according to any one of claims 1 to 3, wherein a zone where an intensity of an electric field generated around the first conductive portion greater than a field intensity threshold of the target tissue is a first ablation zone, a zone where an intensity of an electric field generated around the second conductive portion greater than the field intensity threshold of the target tissue is a second ablation zone, and the first ablation zone and the second ablation zone are partially overlapped with each other.

8. The ablation system according to claim 6 or 7, wherein the first ablation zone and the second ablation zone are formed at different axial positions of the ablation system.

9. The ablation system according to any one of claims 1 to 8, wherein the first conductive portion is provided on a peripheral edge area of the support.

10. The ablation system according to claim 9, wherein the first conductive portion is at least provided at a portion with the maximum radial dimension on the peripheral edge area of the support.

11. The ablation system according to any one of claims 1 to 10, wherein the shortest line between any point on the first conductive portion and any point on the second conductive portion is the shortest electric field line, and at least one shortest electric field line passes through an area outside the support.

12. The ablation system according to claim 11, wherein the support comprises an accommodating part which is adjacent to an axis of the support relative to the first conductive portion and/or the second conductive portion, an accommodating space for accommodating a tissue to be ablated defined outside an outer peripheral wall of the accommodating part, the first conductive portion and the second conductive portion respectively arranged on opposite sides of the accommodating space, and during ablation, at least one shortest electric field line passes through the accommodating space.

13. The ablation system according to claim 12, wherein at least part of an outer wall of the accommodating part is configured to contact the tissue to be ablated.

14. The ablation system according to claim 12 or 13, wherein the accommodating part is arranged in a circumferential direction of the support, and the first conductive portion and the second conductive portion are axially spaced apart from each other.

15. The ablation system according to any one of claims 11 to 14, wherein at least one shortest electric field line passing through the accommodating space is inclined relative to an axis of the support.

16. The ablation system according to claim 15, wherein in a natural state, a radial dimension of a ring structure formed by a proximal conductive portion of the first conductive portion and the second conductive portion is greater than that a radial dimension of a ring structure formed by a distal conductive portion of the first conductive portion and the second conductive portion.

17. The ablation system according to any one of claims 1 to 16, wherein the target tissue is myocardial tissue.

18. The ablation system according to claim 17, wherein the target tissue is left atrial appendage, and the ablation system is configured to occlude and ablate the left atrial appendage, and wherein the support comprises a sealing part and an anchoring part connected to each other, the sealing part is configured to cover an ostium of the left atrial appendage, and the anchoring part is configured to be fixed to a tissue wall of the left atrial appendage.

19. The ablation system according to claim 18, wherein the first conductive portion is provided on a peripheral edge area of the sealing part.

20. The ablation system according to claim 19, wherein the second conductive portion is provided on the support, and the first conductive portion is provided on a proximal side of the second conductive portion.

21. The ablation system according to claim 19, wherein the sealing part is provided on a proximal side of the anchoring part, and the second conductive portion is provided on a peripheral edge area of the anchoring part.

22. The ablation system according to claim 21, wherein the sealing part and the anchoring part are insulated from and connected to each other through a connecting piece.

23. The ablation system according to any one of claims 1 to 18, wherein the second conductive portion is provided on the delivery device, and the second conductive portion is movable relative to the support and configured to be released at a proximal side of the support.

24. The ablation system according to claim 23, wherein a portion of the support with the maximum radial dimension is located between a proximal end and a distal end of the support, or a side wall of the support has the same radial dimension at different axial positions.

25. The ablation system according to any one of claims 1 to 19, wherein the second conductive portion is provided on the delivery device, and the second conductive portion is movable relative to the support and configured to be released at a distal side of the support.

26. The ablation system according to any one of claims 1 to 25, wherein the support is a radially compressible and expandable frame or balloon, and wherein the frame is made by weaving or cutting and has a plurality of meshes, and the balloon is configured to be filled with a medium to adjust a radial size thereof.

27. The ablation system according to any one of claims 1 to 26, wherein at least part of the first conductive portion and/or the second conductive portion is configured to collect physiological signals of the tissue.
